# EUROPEAN PATENT APPLICATION

(11) **EP 4 325 210 A1**
(43) Date of publication of application: **21.02.2024**
(21) Application number: 22788211.5
(22) Date of filing: 14.04.2022
(51) Int. Cl.: G01N 21/78, C09K 11/06

(54) **COMPOSITION, STABILIZATION METHOD, METHOD FOR MEASURING AMOUNT OF LUMINESCENCE, AND STABILIZER FOR LUMINESCENT SUBSTRATE**

(30) Priority: 16.04.2021 JP 2021069924
(71) Applicant: FUJIFILM Corporation, Tokyo 106-8620 (JP)
(72) Inventor: YOSHIDA, Yukihiro, Amagasaki-shi, Hyogo 661-0963 (JP); MATSUDA, Shinjiro, Amagasaki-shi, Hyogo 661-0963 (JP)
(74) Representative: Beacham, Annabel Rose
(86) International application number: PCT/JP2022/017860
(87) International publication number: WO 2022/220288

(57) **Abstract**

The present invention relates to a composition containing a transition metal compound selected from a vanadium compound, a niobium compound, or a tantalum compound, and a luminescent substrate, a stabilization method of a luminescent substrate, a method for measuring an amount of luminescence, and a stabilizer for a luminescent substrate, containing the transition metal compound.

## Description

### Technical Field

The present invention relates to a composition containing a luminescent substrate and a transition metal compound selected from a vanadium compound, a niobium compound, or a tantalum compound, a stabilization method of a luminescent substrate, a method for measuring an amount of luminescence, and a stabilizer for a luminescent substrate.

### Background Art

Since an analysis method based on a chemiluminescence reaction enables high-sensitivity measurement, the analysis method has been actively studied as a measurement system in the immunological field. As a typical measuring method, there is a method of reacting an antigen or an antibody labeled with an enzyme such as a peroxidase with luminol or a luminol derivative such as isoluminol, which is a chemiluminescent substance, and measuring an amount of luminescence. Since it is necessary to detect a target substance with high sensitivity to quantify substances which exist only in a trace amount in a living body, the luminol or the luminol derivative has been widely used as a compound having an excellent chemiluminescence quantum yield.

In the measurement system using the luminol derivative, since an optimum pH of the luminescence is weakly alkaline, as a luminescent reagent containing the luminol derivative, a borate buffer solution has been used (for example, Patent Document 1)

### Prior Art Documents

### Patent Documents

Patent Document 1: JP2008-203088A

### SUMMARY OF THE INVENTION

Meanwhile, since it has been reported that boric acid has reproductive toxicity, it is desirable not to use the boric acid in consideration of the effect on the human body and the environment. However, unexpectedly, the boric acid contributes to stabilization of the luminol derivative, and in a case where the boric acid is not used, the amount of luminescence is reduced with the temporal change, and it has been found that it is difficult to maintain performance of the luminescent reagent in a solution state.

The present invention has been made in consideration of the above-described circumstances, and an object of the present invention is to find a composition capable of improving stability of a luminescent reagent in a solution state, which contains a luminescent substrate such as a luminol derivative, and to provide a stabilized luminescent reagent and the like.

The present invention has the following configurations.
(1) A composition (hereinafter, may be abbreviated as a composition according to the embodiment of the present invention) comprising:
   a transition metal compound selected from a vanadium compound, a niobium compound, or a tantalum compound; and
   a luminescent substrate.
(2) The composition according to (1),
   in which the transition metal compound is a vanadium compound.
(3) The composition according to (1),
   in which the transition metal compound is a compound represented by General Formula (1) or General Formula (2), 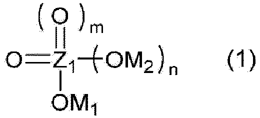 (in General Formula (1), Z₁ represents a vanadium atom, a niobium atom, or a tantalum atom, M₁ represents a hydrogen atom, an alkali metal atom, or NH₄, M₂'s each independently represent a hydrogen atom, an alkali metal atom, or NH₄, m represents 0 or 1, and n represents 2 in a case where m is 0 or represents 0 in a case where m is 1) 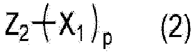 (in General Formula (2), Z₂ represents a vanadium atom, a niobium atom, or a tantalum atom, X₁ represents a hydroxy group, a chlorine atom, or a bromine atom, and p represents an integer of 2 to 5).
(4) The composition according to (3),
   in which the composition contains the compound represented by General Formula (1) and a luminescent substrate.
(5) The composition according to (4),
   in which Z₁ in General Formula (1) is a vanadium atom.
(6) The composition according to (4) or (5),
   in which the compound represented by General Formula (1) is orthovanadic acid, an alkali metal salt of orthovanadic acid, an ammonium salt of orthovanadic acid, metavanadic acid, an alkali metal salt of metavanadic acid, or an ammonium salt of metavanadic acid.
(7) The composition according to any one of (1) to (6),
   in which the luminescent substrate is luminols, a chemically acceptable salt of luminols, 8-amino-5-chloro-7-phenylpyrido[3,4-d]pyridazine-1,4(2H,3H)-dione, or a chemically acceptable salt of 8-amino-5-chloro-7-phenylpyrido[3,4-d]pyridazine-1,4(2H,3H)-dione.
(8) The composition according to any one of (1) to (7),
   in which the transition metal compound is a compound for stabilizing the luminescent substrate.
(9) The composition according to any one of (1) to (8),
   in which a concentration of the transition metal compound in the composition is 0.01 to 10 mM.
(10) The composition according to any one of (1) to (9),
   in which a pH of the composition is 5 to 12.
(11) A stabilization method of a luminescent substrate (hereinafter, may be abbreviated as a stabilization method according to the embodiment of the present invention), comprising:
   coexisting a transition metal compound selected from a vanadium compound, a niobium compound, or a tantalum compound with a luminescent substrate.
(12) The stabilization method according to (11),
   in which the transition metal compound is added to a solution containing the luminescent substrate for the coexistence.
(13) The stabilization method according to (11) or (12),
   in which the transition metal compound is a compound represented by General Formula (1) or General Formula (2), 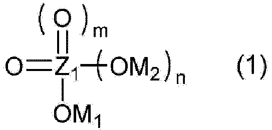 (in General Formula (1), Z₁ represents a vanadium atom, a niobium atom, or a tantalum atom, M₁ represents a hydrogen atom, an alkali metal atom, or NH₄, M₂'s each independently represent a hydrogen atom, an alkali metal atom, or NH₄, m represents 0 or 1, and n represents 2 in a case where m is 0 or represents 0 in a case where m is 1) 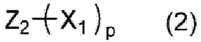 (in General Formula (2), Z₂ represents a vanadium atom, a niobium atom, or a tantalum atom, X₁ represents a hydroxy group, a chlorine atom, or a bromine atom, and p represents an integer of 2 to 5).
(14) A method for measuring an amount of luminescence (hereinafter, may be abbreviated as a measuring method according to the embodiment of the present invention), the method comprising:
   reacting a luminescent substrate with an oxidizing agent and an oxidation catalyst in a presence of a transition metal compound selected from a vanadium compound, a niobium compound, or a tantalum compound.
(15) The method according to (14),
   in which the transition metal compound is a compound represented by General Formula (1) or General Formula (2), 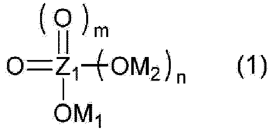 (in General Formula (1), Z₁ represents a vanadium atom, a niobium atom, or a tantalum atom, M₁ represents a hydrogen atom, an alkali metal atom, or NH₄, M₂'s each independently represent a hydrogen atom, an alkali metal atom, or NH₄, m represents 0 or 1, and n represents 2 in a case where m is 0 or represents 0 in a case where m is 1) 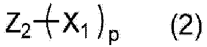 (in General Formula (2), Z₂ represents a vanadium atom, a niobium atom, or a tantalum atom, X₁ represents a hydroxy group, a chlorine atom, or a bromine atom, and p represents an integer of 2 to 5).
(16) A stabilizer for a luminescent substrate, comprising:
   a transition metal compound selected from a vanadium compound, a niobium compound, or a tantalum compound.

In the composition and stabilization method according to the embodiment of the present invention, stability of the luminescent substrate can be improved by coexisting the transition metal compound selected from a vanadium compound, a niobium compound, or a tantalum compound with the luminescent substrate. As a result, it is possible to suppress a decrease in an amount of luminescence of the luminescent substrate (decrease in measurement sensitivity) due to decomposition, deterioration, or the like of the luminescent substrate.

The measuring method according to the embodiment of the present invention is a method for measuring an amount of luminescence derived from a target substance (substance to be measured) by reacting the luminescent substrate with the oxidation catalyst in the presence of the transition metal compound selected from a vanadium compound, a niobium compound, or a tantalum compound, in which stability of the luminescent substrate can be improved during the reaction and the measurement of the amount of luminescence, and the amount of luminescence derived from the target substance can be measured with high sensitivity and high accuracy.

### BRIEF DESCRIPTION OF THE DRAWINGS

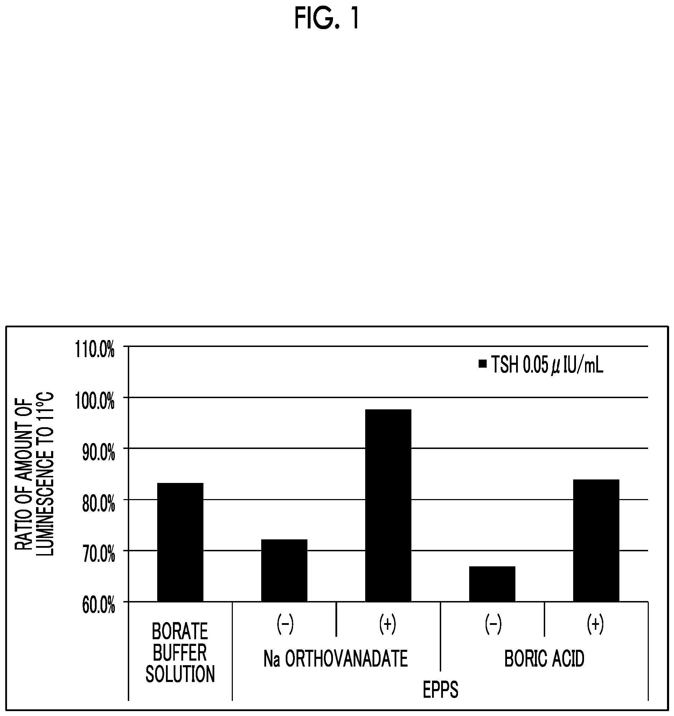

Fig. 1 is a graph showing a ratio of an amount of luminescence to 11°C in a case where a standard TSH solution with a TSH concentration of 0.05 µIU/mL is used in results of Table 1 (Example 1 and Comparative Examples 1 and 2).

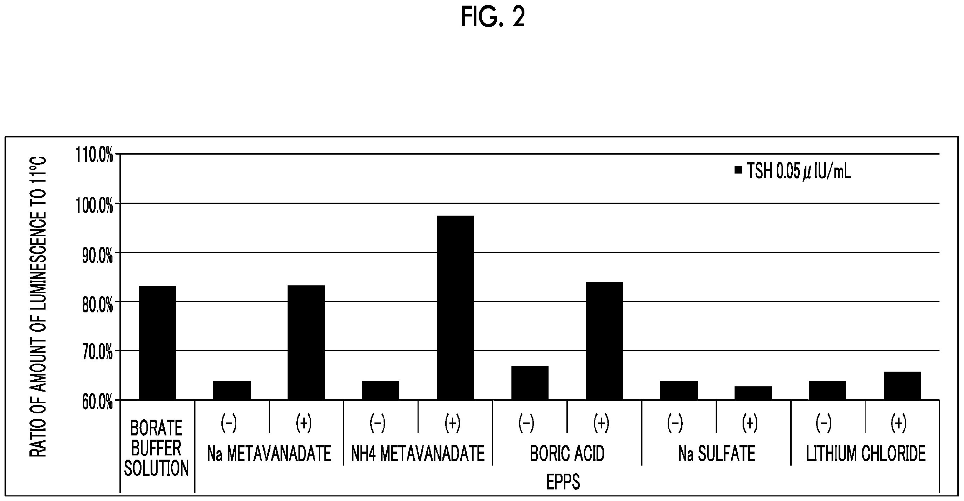

Fig. 2 is a graph showing a ratio of an amount of luminescence to 11°C in a case where a standard TSH solution with a TSH concentration of 0.05 µIU/mL is used in results of Table 2 (Examples 2 and 3 and Comparative Examples 1 to 4).

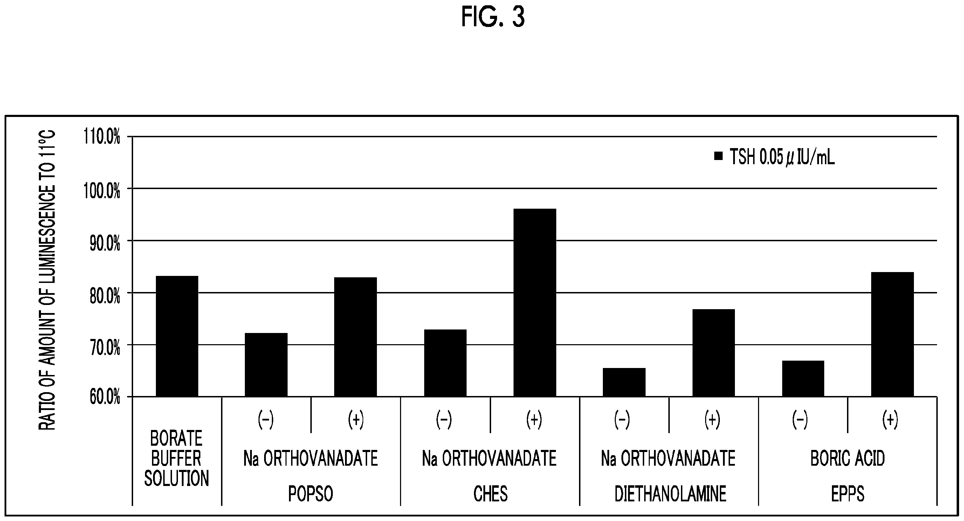

Fig. 3 is a graph showing a ratio of an amount of luminescence to 11°C in a case where a standard TSH solution with a TSH concentration of 0.05 µIU/mL is used in results of Table 3 (Examples 4 to 6 and Comparative Examples 1 and 2).

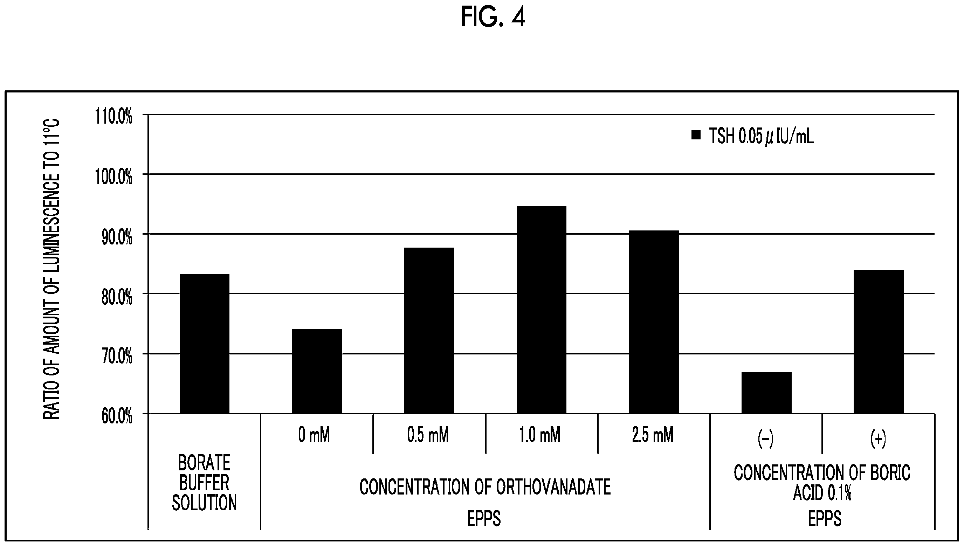

Fig. 4 is a graph showing a ratio of an amount of luminescence to 11°C in a case where a standard TSH solution with a TSH concentration of 0.05 µIU/mL is used in results of Table 4 (Examples 7 to 9 and Comparative Examples 1 and 2).

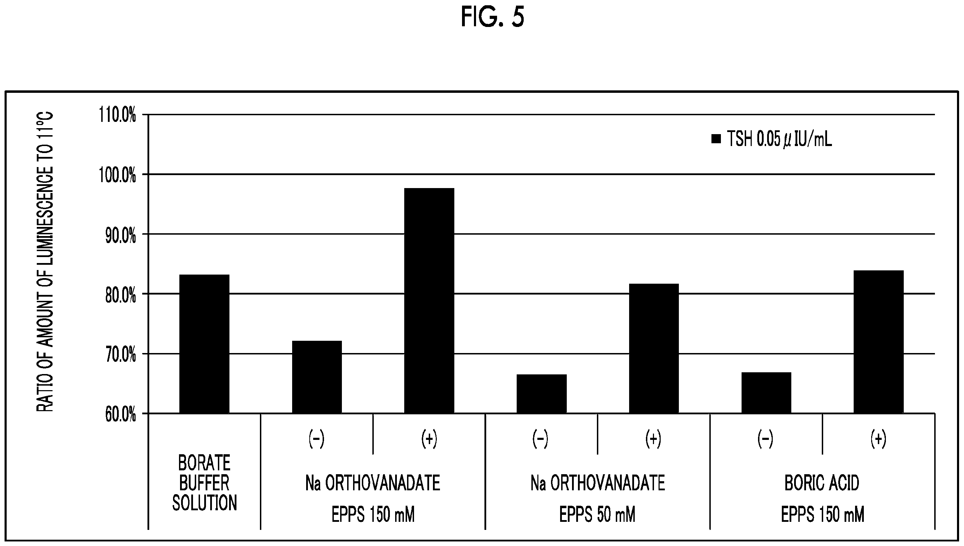

Fig. 5 is a graph showing a ratio of an amount of luminescence to 11°C in a case where a standard TSH solution with a TSH concentration of 0.05 µIU/mL is used in results of Table 5 (Examples 1 and 10 and Comparative Examples 1 and 2).

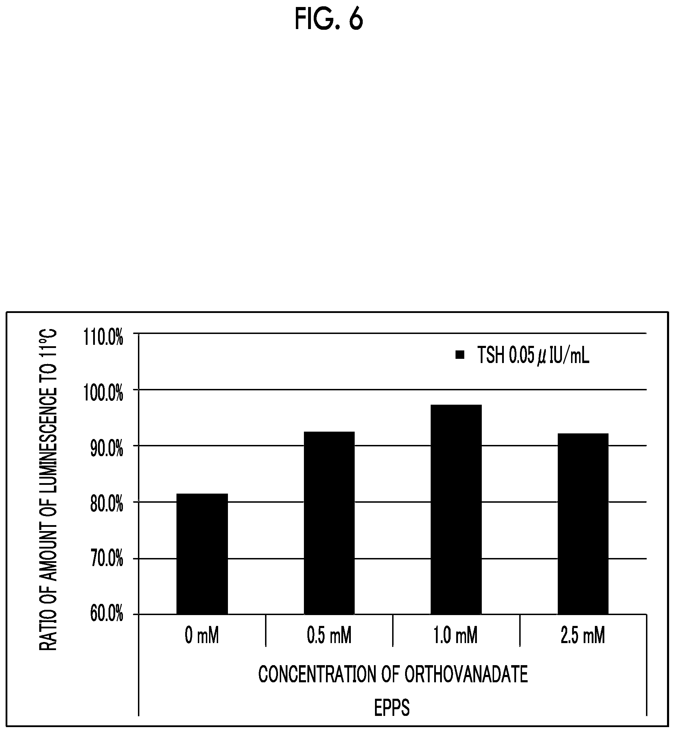

Fig. 6 is a graph showing a ratio of an amount of luminescence to 11°C in a case where a standard TSH solution with a TSH concentration of 0.05 µIU/mL is used in results of Table 6 (Examples 11 to 13).

### DESCRIPTION OF THE PREFERRED EMBODIMENTS

Hereinafter, the present invention will be described in detail. In the present specification, "and/or" is used to mean either one or both. "to" representing a numerical value range means a range including values of the upper limit and the lower limit. All publications and patents cited in the section of the detailed description of the invention in the present specification constitute a part of the present specification by, for example, indicating methods, materials, and the like, which can be used in the present invention.

In addition, in the present specification, "mM" is synonymous with "mmol/l".

### -Composition according to embodiment of present invention-

The composition according to the embodiment of the present invention contains a transition metal compound selected from a vanadium compound, a niobium compound, or a tantalum compound (hereinafter, may be abbreviated as a transition metal compound according to the present invention), and a luminescent substrate. During storage or use (during measurement) of the composition according to the embodiment of the present invention, decomposition or deterioration of the luminescent substrate is suppressed by action of the transition metal compound. For example, in a case where the composition according to the embodiment of the present invention is used in chemiluminescence measurement, it is possible to measure a target substance in a living body with high sensitivity and high accuracy.

The transition metal compound according to the present invention refers to a vanadium compound having a vanadium element in the compound, a niobium compound having a niobium element in the compound, or a tantalum compound having a tantalum element in the compound. Preferred specific examples of such a transition metal compound include a compound represented by General Formula (1) or General Formula (2). 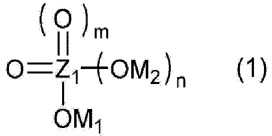 (in General Formula (1), Z₁ represents a vanadium atom, a niobium atom, or a tantalum atom, M₁ represents a hydrogen atom, an alkali metal atom, or NH₄, M₂'s each independently represent a hydrogen atom, an alkali metal atom, or NH₄, m represents 0 or 1, and n represents 2 in a case where m is 0 or represents 0 in a case where m is 1) 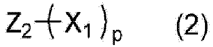 (in General Formula (2), Z₂ represents a vanadium atom, a niobium atom, or a tantalum atom, X₁ represents a hydroxy group, a chlorine atom, or a bromine atom, and p represents an integer of 2 to 5)

In General Formula (1), examples of the alkali metal atom represented by M₁ and M₂ include a lithium atom, a sodium atom, a potassium atom, and a cesium atom, and among these, a lithium atom, a sodium atom, or a potassium atom is preferable, and a sodium atom or a potassium atom is more preferable.

As Z₁ in General Formula (1) and Z₂ in General Formula (2), a vanadium atom or a tantalum atom is preferable, and a vanadium atom is more preferable.

As M₁ and M₂ in General Formula (1), an alkali metal atom or NH₄ is preferable, and NH₄ is more preferable.

In General Formula (1), M₂'s may be the same group or different groups.

m in General Formula (1) is preferably 0. A case where m is 0 means that there is no (=O)m group.

A case where n in General Formula (1) is 0 means that there is no (-OM₂)ₙ group.

As X₁ in General Formula (2), a chlorine atom or a bromine atom is preferable, and a chlorine atom is more preferable.

p in General Formula (1) is preferably an integer of 3 to 5, more preferably 3 or 5, and still more preferably 3.

Specific examples of such compounds represented by General Formulae (1) and (2) include orthovanadic acid (V) (H₃VO₄); alkali metal salts of orthovanadic acid, such as lithium orthovanadate (V) (Li₃VO₄), sodium orthovanadate (V) (Na₃VO₄), potassium orthovanadate (V) (K₃VO₄), and cesium orthovanadate (V) (Cs₃VO₄); ammonium salts of orthovanadic acid, such as ammonium orthovanadate (V) (NH₄VO₄); metavanadic acid (V) (HVO₃); alkali metal salts of metavanadic acid, such as lithium metavanadate (V) (LiVO₃), sodium metavanadate (V) (NaVO₃), potassium metavanadate (V) (KVO₃), and cesium metavanadate (V) (CsVO₃); ammonium salts of metavanadic acid, such as ammonium metavanadate (V) (NH₄VO₃);orthoniobic acid (V) (H₃NbO₄); alkali metal salts of orthoniobic acid, such as lithium orthoniobate (V) (Li₃NbO₄), sodium orthoniobate (V) (Na₃NbO₄), potassium orthoniobate (V) (K₃NbO₄), and cesium orthoniobate (V) (Cs₃NbO₄); ammonium salts of orthoniobic acid, such as ammonium orthoniobate (V) (NH₄NbO₄); metaniobic acid (V) (HNbO₃); alkali metal salts of metaniobic acid, such as lithium metaniobate (V) (LiNbO₃), sodium metaniobate (V) (NaNbO₃), potassium metaniobate (V) (KNbO₃), and cesium metaniobate (V) (CsNbO₃); ammonium salts of metaniobic acid, such as ammonium metaniobate (V) (NH₄NbO₃);orthotantalic acid (V) (H₃TaO₄); alkali metal salts of orthotantalic acid, such as lithium orthotantalate (V) (Li₃TaO₄), sodium orthotantalate (V) (Na₃TaO₄), potassium orthotantalate (V) (K₃TaO₄), and cesium orthotantalate (V) (Cs₃TaO₄); ammonium salts of orthotantalic acid, such as ammonium orthotantalate (V) (NH₄TaO₄); metatantalic acid (V) (HTaO₃); alkali metal salts of metatantalic acid, such as lithium metatantalate (V) (LiTaO₃), sodium metatantalate (V) (NaTaO₃), potassium metatantalate (V) (KTaO₃), and cesium metatantalate (V) (CsTaO₃); ammonium salts of metatantalic acid, such as ammonium metatantalate (V) (NH₄TaO₃);vanadium (II) hydroxide (V(OH)₂); vanadium halides such as vanadium (III) chloride (VCl₃), vanadium (IV) chloride (VCl₄), vanadium (V) chloride (VCl₅), vanadium (III) bromide (VBr₃), vanadium (IV) bromide (VBr₄), and vanadium (V) bromide (VBr₅); niobium (V) hydroxide (Nb(OH)s); niobium halides such as niobium (III) chloride (NbCl₃), niobium (IV) chloride (NbCl₄), niobium (V) chloride (NbCl₅), niobium (III) bromide (NbBr₃), niobium (IV) bromide (NbBr₄), and niobium (V) bromide (NbBr₅); tantalum (V) hydroxide (Ta(OH)₅); and tantalum halides such as tantalum (III) chloride (TaCl₃), tantalum (IV) chloride (TaCl₄), tantalum (V) chloride (TaCl₅), tantalum (III) bromide (TaBr₃), tantalum (IV) bromide (TaBr₄), and tantalum (V) bromide (TaBrs). These compounds may form a complex with an organic solvent such as tetrahydrofuran. In addition, as these compounds, commercially available products may be used, and those appropriately synthesized by a known method may be used.

Among the transition metal compounds according to the present invention, the above-described compound represented by General Formula (1) is preferable, and a compound represented by General Formula (1-1) is more preferable. 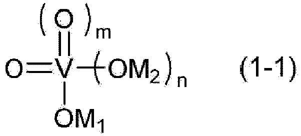 (in General Formula (1-1), M₁, M₂, m, and n are the same as M₁, M₂, m, and n in Formula (1) described above)

Specific examples of such a compound represented by General Formula (1-1) include orthovanadic acid (V) (H₃VO₄), an alkali metal salt of orthovanadic acid, an ammonium salt of orthovanadic acid, metavanadic acid (V) (HVO₃), an alkali metal salt of metavanadic acid, and an ammonium salt of metavanadic acid. Among these, orthovanadic acid (V) (H₃VO₄), sodium orthovanadate (V) (Na₃VO₄), potassium orthovanadate (V) (K₃VO₄), ammonium orthovanadate (V) (NH₄VO₄), metavanadic acid (V) (HVO₃), sodium metavanadate (V) (NaVO₃), potassium metavanadate (V) (KVO₃), or ammonium metavanadate (V) (NH₄VO₃) is preferable; sodium orthovanadate (V) (Na₃VO₄), potassium orthovanadate (V) (K₃VO₄), ammonium orthovanadate (V) (NH₄VO₄), sodium metavanadate (V) (NaVO₃), potassium metavanadate (V) (KVO₃), or ammonium metavanadate (V) (NH₄VO₃) is more preferable; and sodium orthovanadate (V) (Na₃VO₄), ammonium orthovanadate (V) (NH₄VO₄), sodium metavanadate (V) (NaVO₃), or ammonium metavanadate (V) (NH₄VO₃) is still more preferable. Among the transition metal compounds according to the present invention, these preferred vanadic acids have effects that it is less likely to adversely affect measurement results during the measurement, it is possible to more effectively improve the stability of the luminescent substrate, and it is possible to measure the target object with high accuracy.

In the composition according to the embodiment of the present invention, as the transition metal compound, one kind of compound may be used alone, or two or more kinds of compounds may be used in combination.

The luminescent substrate contained in the composition according to the embodiment of the present invention refers to a luminescent substance used for measuring chemiluminescence of the target substance, and is not particularly limited as long as it is a luminescent substrate (luminescent substance) used in the related art. In the chemiluminescence measurement, the presence or absence of the target substance or the amount of the target substance is detected by detecting chemiluminescence emitted from a chemiluminescent substance generated in a case where the luminescent substrate (luminescent substance) is decomposed by a labeling enzyme. Among such luminescent substrates, a substrate which emits light by radicalization of the luminescent substrate is preferable, and specific examples thereof include luminols such as luminol, isoluminol, N-ethylisoluminol, N-(4-aminobutyl)-N-ethylisoluminol hemisuccimide, and N-(6-aminohexyl)-N-ethylisoluminol; chemically acceptable salts of luminols (alkali metal salts such as a sodium salt and a potassium salt; alkaline earth metal salts such as a magnesium salt and a calcium salt; ammonium salts; and the like); 8-amino-5-chloro-7-phenylpyrido[3,4-d]pyridazine-1,4(2H,3H)-dione (L-012) or chemically acceptable salts of L-012; lucigenin; luciferin (firefly luciferin, bacterial luciferin, and coelenterazine); and bis(2,4,6-trichlorophenyl)oxalate. Specific examples of the chemically acceptable salts of L-012 include salts described in WO2017/069192A. Among these luminescent substrates, luminols or chemically acceptable salts thereof are preferable, luminol, isoluminol, or chemically acceptable salts thereof are more preferable, and luminol or chemically acceptable salts of luminol are still more preferable. As these luminescent substrates, commercially available products may be used, and those appropriately synthesized by a known method may be used.

The composition according to the embodiment of the present invention may be in a solution state, may be in a frozen state, or may be in a freeze-dried state.

In a case where the composition according to the embodiment of the present invention is in a frozen state or in a freeze-dried state, it is preferable to use a dissolving solution for dissolving the transition metal compound according to the present invention and the luminescent substrate according to the present invention. Such a dissolving solution is not particularly limited as long as it is a dissolving solution used in the related art, and specific examples thereof include water and a buffer solution.

The buffer solution according to the composition method of the present invention is not particularly limited as long as it is a buffer solution used in the related art, and examples thereof include buffer solutions in a buffer agent such as N-(2-acetoamido)-2-aminoethanesulfonic acid (ACES), N-(2-acetoamido)iminodiacetic acid (ADA), N,N-bis(2-hydroxyethyl)-2-aminoethanesulfonic acid (BES), N,N-bis(2-hydroxyethyl)glycine (Bicine), bis(2-hydroxyethyl)iminotris(hydroxyethyl)methane (Bis-Tris), N-cyclohexyl-3-aminopropanesulfonic acid (CAPS), N-cyclohexyl-2-hydroxy-3-aminopropanesulfonic acid (CAPSO), N-cyclohexyl-2-aminoethanesulfonic acid (CHES), 3-[N,N-bis(2-hydroxyethyl)amino]-2-hydroxypropanesulfonic acid (DIPSO), 3-[4-(2-hydroxyethyl)-1-piperazinyl]propanesulfonic acid (EPPS), 2-[4-(2-hydroxyethyl)-1-piperazinyl]ethanesulfonic acid (HEPES), 2-hydroxy-3-[4-(2-hydroxyethyl)-1-piperazinyl]propanesulfonic acid (HEPPSO), 2-(N-morpholino)ethanesulfonic acid (MES), 3-(N-morpholino)propanesulfonic acid (MOPS), 2-hydroxy-3-(N-morpholino)propanesulfonic acid (MOPSO), piperazine-1,4-bis(2-ethanesulfonic acid) (PIPES), piperazine-1,4-bis(2-hydroxy-3-propanesulfonic acid) (POPSO), N-tris(hydroxymethyl)methyl-3-aminopropanesulfonic acid (TAPS), 2-hydroxy-N-tris(hydroxymethyl)methyl-3-aminopropanesulfonic acid (TAPSO), N-tris(hydroxymethyl)methyl-2-aminoethanesulfonic acid (TES), and N-[tris(hydroxymethyl)methyl]glycine (Tricine), or a salt thereof is dissolved; buffer solutions in which a buffer agent such as phosphoric acid, carbonic acid, acetic acid, citric acid, and tartaric acid, or a salt thereof is dissolved; buffer solutions in which a buffer agent such as tris(hydroxymethyl)aminomethane (Tris), diethanolamine, and triethanolamine is dissolved; and veronal buffer solutions. Among such buffer solutions, a buffer solution in which N-cyclohexyl-2-aminoethanesulfonic acid (CHES), 3-[4-(2-hydroxyethyl)-1-piperazinyl]propanesulfonic acid (EPPS), piperazine-1,4-bis(2-hydroxy-3-propanesulfonic acid) (POPSO), tris(hydroxymethyl)aminomethane (Tris), diethanolamine, or salts thereof are dissolved is preferable; and a buffer solution in which N-cyclohexyl-2-aminoethanesulfonic acid (CHES), 3-[4-(2-hydroxyethyl)-1-piperazinyl]propanesulfonic acid (EPPS), piperazine-1,4-bis(2-hydroxy-3-propanesulfonic acid) (POPSO), tris(hydroxymethyl)aminomethane (Tris), or salts thereof are dissolved is more preferable. In addition, as these buffer solutions, one kind of buffer solution may be used alone, or two or more kinds of buffer solutions may be used in combination; and these buffer solutions may be used in combination with alkali metal hydroxides such as sodium hydroxide and potassium hydroxide or acids such as hydrochloric acid and sulfuric acid. Specific examples of the combination of the buffer solution include a buffer solution of N-cyclohexyl-2-aminoethanesulfonic acid (CHES) and sodium hydroxide, a buffer solution of 3-[4-(2-hydroxyethyl)-1-piperazinyl]propanesulfonic acid (EPPS) and sodium hydroxide, a buffer solution of piperazine-1,4-bis(2-hydroxy-3-propanesulfonic acid) (POPSO) and sodium hydroxide, and a buffer solution of tris(hydroxymethyl)aminomethane (Tris) and sodium hydroxide. As these buffer solutions, commercially available products may be used, and those appropriately prepared by a known method may be used.

A concentration of the buffer agent in the buffer solution in the composition according to the embodiment of the present invention may be appropriately selected from a range of usually 10 to 500 mM, preferably 10 to 300 mM.

A concentration of the luminescent substrate in the composition according to the embodiment of the present invention may be appropriately selected from a concentration range used in the field of chemiluminescence measurement, and specifically, a lower limit of the concentration of the luminescent substrate in the solution is usually 0.01 mM or more, preferably 0.1 mM or more and more preferably 0.2 mM or more, and an upper limit thereof is usually 20 mM or less, preferably 10 mM or less and more preferably 5 mM or less.

A concentration of the transition metal compound in the composition according to the embodiment of the present invention is not particularly limited as long as it can improve the stability of the luminescent substrate. For example, the above-described concentration varies depending on the type of the transition metal compound according to the present invention, the concentration of the coexisting luminescent substrate, and the like, and for example, in a case where the concentration of the luminescent substrate is used as a standard, a lower limit of the concentration of the transition metal compound in a solution with respect to the concentration of 1 mM of the luminescent substrate in the solution is usually 0.0005 mM or more, preferably 0.005 mM or more, more preferably 0.05 mM or more, and still more preferably 0.1 mM or more, and an upper limit thereof is usually 10 mM or less, preferably 5 mM or less, more preferably 2.5 mM or less, and still more preferably 1 mM or less. In addition, a lower limit of the concentration of the transition metal compound in a solution is usually 0.01 mM or more, preferably 0.05 mM or more, more preferably 0.1 mM or more, still more preferably 0.25 mM or more, and most preferably 0.5 mM or more, and an upper limit thereof is usually 10 mM or less, preferably 5 mM or less, more preferably 2.5 mM or less, and still more preferably 1 mM or less.

A pH of the composition according to the embodiment of the present invention may be appropriately selected from a range of usually pH 5 to 12, preferably pH 6 to 11 and more preferably pH 6.5 to 10.5.

In a case where the composition according to the embodiment of the present invention is in a solution state, it is desirable to add the transition metal compound according to the present invention and the luminescent substrate to the above-described dissolving solution so that the concentration range and concentration ratio described above are satisfied. In addition, in a case where the composition according to the embodiment of the present invention is in a frozen state or in a freeze-dried state, it is desirable that a frozen stock solution or a freeze-dried stock solution contains the transition metal compound according to the present invention and the luminescent substrate with the concentration range and concentration ratio described above. In a case of preparing the composition according to the embodiment of the present invention with the dissolving solution, such as the case of dissolving the composition according to the embodiment of the present invention in a frozen state or in a freeze-dried state with the dissolving solution and the case of diluting the composition according to the embodiment of the present invention in a solution state with the dissolving solution, the stability of the luminescent substrate in the prepared composition can be improved by preparing the transition metal compound according to the present invention and the luminescent substrate after the preparation (dissolution, dilution, and the like) with the concentration range and concentration ratio described above.

In addition to the transition metal compound according to the present invention and the luminescent substrate, water, and/or the buffer solution, the composition according to the embodiment of the present invention may contain an additive used in the related art, for example, salts such as sodium chloride and magnesium chloride; preserving agents such as sodium azide; sugars such as maltose, sucrose, and trehalose; preservatives such as 5-chloro-2-methyl-4-isothiazolin-3-one, 2-methyl-4-isothiazolin-3-one, 5-bromo-5-nitro-1,3-dioxane, and (+)-10-camphorsulfonic acid; stabilizers; sensitizers; surfactants; proteins; and the like. As these additives, commercially available products may be used, and those appropriately synthesized by a known method may be used. In addition, it is desirable that the composition according to the embodiment of the present invention does not contain boric acid or a salt thereof.

Specific examples of the above-described stabilizer include those represented by Formulae [3-1] to [3-12]. As these stabilizers, one kind of stabilizer may be used alone, or two or more kinds of stabilizers may be used in combination. The above-described stabilizer is not limited to those represented by Formulae [3-1] to [3-12].

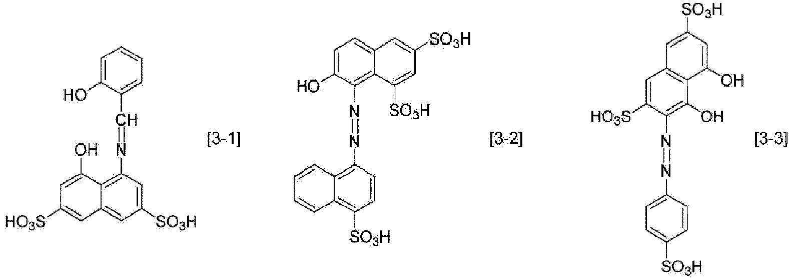

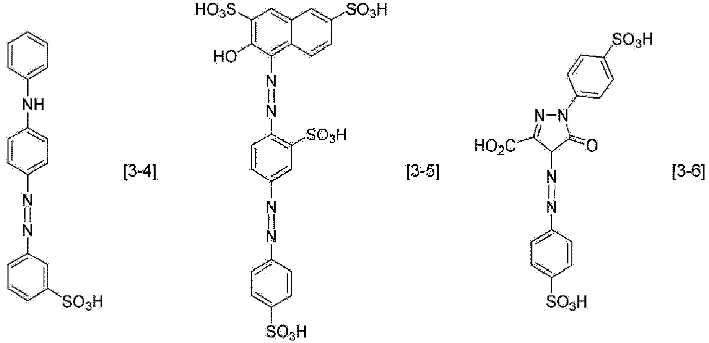

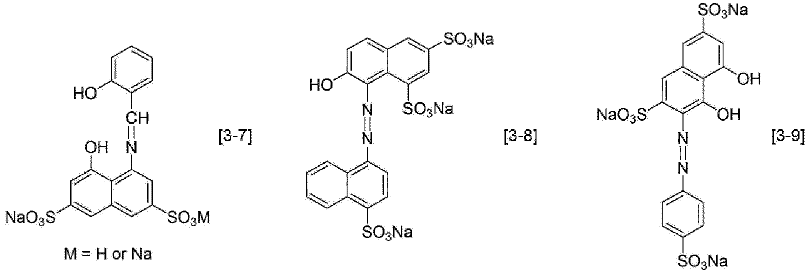

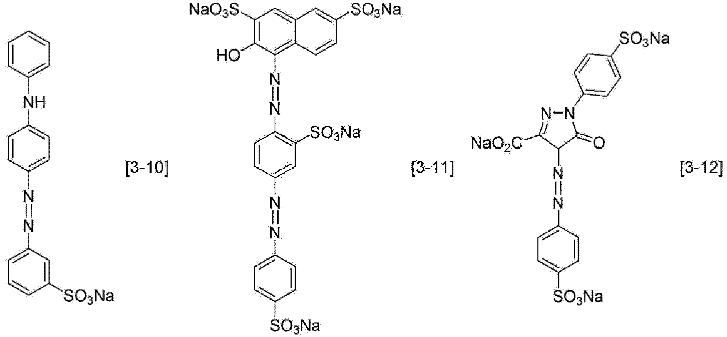

Specific examples of the above-described preferred stabilizers and specific examples of stabilizers other than [3-1] to [3-12] include compounds or salts thereof described in WO2017/069192A.

The above-described sensitizer (enhancer) is not particularly limited as long as it is a sensitizer (enhancer) used in the related art, and specific examples thereof include thiazole derivatives such as 4-(4-hydroxyphenyl)thiazole; imidazole derivatives such as 4-(imidazol-1-yl)phenol; oxazole derivatives such as 4-(4-hydroxyphenyl)oxazole; phenol derivatives such as p-iodophenol, 4-hydroxycinnamic acid, and 4-(4'-thiazolyl)phenol; and naphthol derivatives such as 1-bromonaphthol. Among these, thiazole derivatives such as 4-(4-hydroxyphenyl)thiazole are preferable. As these sensitizers (enhancers), one kind of sensitizer (enhancer) may be used alone, or two or more kinds of sensitizers (enhancers) may be used in combination.

An amount of these additives added may be appropriately set from a range used in the related art.

The composition according to the embodiment of the present invention may contain an organic solvent for the purpose of improving solubility of the above-described additives in water or the buffer agent. Specific examples of such an organic solvent include alcohol-based solvents such as methanol and ethanol; ether-based solvents such as tetrahydrofuran and 1,4-dioxane; ketone-based solvents such as acetone and ethyl methyl ketone: amide-based solvents such as dimethylformamide and diethylformamide; sulfoxide-based solvents such as dimethyl sulfoxide and diethyl sulfoxide; and nitrile-based solvents such as acetonitrile. As these organic solvents, one kind of organic solvent may be used alone, or two or more kinds of organic solvents may be used in combination. In addition, an amount of these organic solvents used may be appropriately set in consideration of the solubility in water or the buffer solution, the adverse effect on the chemiluminescence measurement described later, and the like.

In the composition according to the embodiment of the present invention, by coexisting the transition metal compound according to the present invention with the luminescent substrate, the stability of the luminescent substrate in a solution state can be improved. Since the luminescent substrate is stable, in the chemiluminescence measurement, it is possible to suppress a decrease in an amount of luminescence of the luminescent substrate (decrease in measurement sensitivity) due to decomposition, deterioration, or the like of the luminescent substrate, and to detect and measure the target substance with high accuracy.

### -Stabilization method according to embodiment of present invention-

The stabilization method according to the embodiment of the present invention is a method of coexisting a transition metal compound selected from a vanadium compound, a niobium compound, or a tantalum compound with a luminescent substrate.

A specific method of the stabilization method according to the embodiment of the present invention is not particularly limited as long as it is a method in which the transition metal compound according to the present invention coexists with the luminescent substrate, and examples thereof include (1) a method of adding the transition metal compound to a solution containing the luminescent substrate so that the transition metal compound coexists with the luminescent substrate, (2) a method of adding the luminescent substrate to a solution containing the transition metal compound so that the transition metal compound coexists with the luminescent substrate, and (3) a method in which the luminescent substrate and the transition metal compound are added to a dissolving solution such as water and an appropriate buffer solution to coexist the transition metal compound with the luminescent substrate.

Examples of the luminescent substrate and the transition metal compound according to the present invention used in the stabilization method according to the embodiment of the present invention include the same ones as in specific examples of the luminescent substrate and the transition metal compound used in the composition according to the embodiment of the present invention described above, and the used concentration and the concentration ratio of the luminescent substrate and the transition metal compound are also the same as those described above.

Examples of the dissolving solution used in the stabilization method according to the embodiment of the present invention include the same ones as in specific examples of the buffer solution used in the composition according to the embodiment of the present invention described above, and preferred specific examples thereof are also the same. In addition, the used concentration of the buffer agent contained in the above-described buffer solution is also the same as the above-described range.

In the stabilization method according to the embodiment of the present invention, examples of the pH of the solution in which the transition metal compound coexists with the luminescent substrate include the same range as the ranges of the pH of the composition according to the embodiment of the present invention, and preferred ranges thereof are also the same.

In the stabilization method according to the embodiment of the present invention, in addition to the transition metal compound according to the present invention, the luminescent substrate, and the dissolving solution, an additive or an organic solvent used in the related art may coexist. Examples of such additives and organic solvents include the same ones as in specific examples of the additives and organic solvents in the composition according to the embodiment of the present invention described above, and the amount of additive added and the amount of organic solvent used are also as described above.

The stabilization method according to the embodiment of the present invention, which is carried out based on the above-described specific method, is a method capable of suppressing decomposition (deterioration) of the luminescent substrate to stabilize the luminescent substrate for a long period of time, thereby suppressing the decrease in amount of luminescence of the luminescent substrate (decrease in measurement sensitivity). In addition, in a case where the luminescent substrate is stabilized by the stabilization method according to the embodiment of the present invention, in using the solution containing the luminescent substrate, the transition metal compound according to the present invention is less likely to adversely affect the measurement, so that the target substance can be measured with high sensitivity and high accuracy.

### -Measuring method according to embodiment of present invention-

The measuring method according to the embodiment of the present invention is a method of reacting a luminescent substrate with an oxidizing agent and an oxidation catalyst in the presence of the transition metal compound according to the present invention. That is, the measuring method according to the embodiment of the present invention is a method of measuring a luminescence reaction of the luminescent substrate and an amount of luminescence of a chemiluminescent substance generated by the luminescence reaction. By using the measuring method according to the embodiment of the present invention, it is possible to detect the presence or absence of the target substance and the amount of the target substance contained in a sample.

Specific examples of the measuring method according to the embodiment of the present invention include various known methods such as a method of detecting and measuring chemiluminescence generated by a reaction (chemiluminescence reaction) between the oxidizing agent and the luminescent substrate.

In the measuring method according to the embodiment of the present invention, as the specific method of reacting the luminescent substrate with an oxidizing agent and an oxidation catalyst, for example, (1) a method of mixing a solution (luminescent substrate solution) containing the luminescent substrate and the transition metal compound according to the present invention, a solution containing the oxidizing agent, and a solution containing the oxidation catalyst in this order for the reaction, (2) a method of mixing a solution (luminescent substrate solution) containing the luminescent substrate and the transition metal compound according to the present invention, a solution containing the oxidation catalyst, and a solution containing the oxidizing agent in this order for the reaction, (3) a method of mixing a solution containing the oxidation catalyst, a solution (luminescent substrate solution) containing the luminescent substrate and the transition metal compound according to the present invention, and a solution containing the oxidizing agent in this order for the reaction, and (4) a method of mixing a solution containing the oxidation catalyst, a solution (luminescent substrate solution) containing the luminescent substrate and the transition metal compound according to the present invention, and a solution containing the oxidizing agent simultaneously for the reaction, or the like is used. These reaction are usually carried out in a solvent.

Examples of the luminescent substrate and the transition metal compound according to the present invention used in the measuring method according to the embodiment of the present invention include the same ones as in specific examples of the luminescent substrate and the transition metal compound used in the composition according to the embodiment of the present invention described above.

The above-described oxidation catalyst refers to a catalyst including a metal such as iron, cobalt, manganese, nickel, chromium, zinc, osmium, molybdenum, cadmium, and copper, and it is not particularly limited as long as it is an oxidation catalyst used in the related art. Specific examples of such an oxidation catalyst include oxidation enzymes such as peroxidase (POD) and catalase; hydrolytic enzymes such as alkaline phosphatase; metalloporphyrin-type compounds such as cobalamin and hematin; hemoproteins such as hemoglobin; and potassium ferricyanide (K₃[Fe(CN)₆]), and among these, oxidation enzymes such as peroxidase (POD) and catalase are preferable, and peroxidase (POD) is more preferable.

The above-described peroxidase (POD) is not particularly limited as long as it can cause the luminescent substrate to undergo chemiluminescence in the presence of an oxidizing agent such as hydrogen peroxide. Specific examples thereof include peroxidases derived from plants such as horseradish, pineapple, and fig (horseradish peroxidase and the like); peroxidases derived from microorganisms such as molds and yeasts; and peroxidases derived from animal leukocytes, thyroid, or the like (myeloperoxidase and the like), and among these, peroxidases derived from horseradish (horseradish peroxidase) are preferable. Such a peroxidase (POD) also includes those produced by genetic engineering or those obtained by hydrolyzing a part of a structure of naturally occurring peroxidase (POD) with a proteolytic enzyme or the like, which have POD activity. In addition, the peroxidase (POD) may be coupled to an antigen, an antibody, or the like.

Examples of the above-described oxidizing agent include inorganic peroxides such as hydrogen peroxide, sodium peroxide, potassium peroxide, perchloric acid, sodium perchlorate, potassium perchlorate, perbromic acid, sodium perbromate, potassium perbromate, sodium permanganate, potassium permanganate, and iodine; and organic peroxides such as peracetic acid, perpropionic acid, peroxomonocarbonic acid, peroxodicarbonic acid, triethylamine-N-oxide, methyldiethylamine-N-oxide, and phthaloyl peroxide, and among these, inorganic peroxides are preferable, hydrogen peroxide or sodium peroxide is more preferable, and hydrogen peroxide is still more preferable. As these oxidizing agents, a commercially available oxidizing agent may be used.

In the measuring method according to the embodiment of the present invention, a sensitizer (enhancer) may be used in addition to the luminescent substrate, the transition metal compound according to the present invention, the oxidation catalyst, and the oxidizing agent.

Examples of the above-described sensitizer (enhancer) include the same one as in specific examples of the sensitizer (enhancer) used in the composition according to the embodiment of the present invention described above, and preferred specific examples thereof are also the same.

An amount of the luminescent substrate used in the measuring method according to the embodiment of the present invention may vary depending on the method of measurement and cannot be generalized, but for example, in a case of measuring total amount of luminescence, with respect to 25 µL of a sample, the amount of the luminescent substrate used is usually 5 µL to 1 mL at a concentration of 0.01 to 20 mM, preferably 10 to 500 µL at a concentration of 0.1 to 10 mM and more preferably 20 to 300 µL at a concentration of 0.2 to 5 mM. On the other hand, in a case of measuring the amount of luminescence per unit time, with respect to 25 µL of a sample, the amount of the luminescent substrate used is usually 5 µL to 1 mL at a concentration of 0.01 to 20 mM, preferably 10 to 500 µL at a concentration of 0.1 to 10 mM and more preferably 20 to 300 µL at a concentration of 0.2 to 5 mM.

An amount of the transition metal compound according to the present invention used in the measuring method according to the embodiment of the present invention may vary depending on the method of measurement and cannot be generalized, but for example, in a case of measuring total amount of luminescence, with respect to 25 µL of a sample, the amount of the luminescent substrate used is usually 5 µL to 1 mL at a concentration of 0.01 to 10 mM, preferably 10 to 500 µL at a concentration of 0.05 to 5 mM, more preferably 20 to 300 µL at a concentration of 0.1 to 2 mM, and still more preferably 20 to 300 µL at a concentration of 0.25 to 1 mM. On the other hand, in a case of measuring the amount of luminescence per unit time, with respect to 25 µL of a sample, the amount of the transition metal compound according to the present invention used is usually 5 µL to 1 mL at a concentration of 0.01 to 10 mM, preferably 10 to 500 µL at a concentration of 0.05 to 5 mM, more preferably 20 to 300 µL at a concentration of 0.1 to 2 mM, and still more preferably 20 to 300 µL at a concentration of 0.25 to 1 mM.

In a solution (luminescent substrate solution) containing the luminescent substrate and the transition metal compound according to the present invention, as a concentration of the transition metal compound to a concentration of the luminescent substrate (concentration ratio of the transition metal compound to the luminescent substrate), with respect to a concentration of 1 mM of the luminescent substrate in the solution, a lower limit of the concentration of the transition metal compound in the solution is usually 0.0005 mM or more, preferably 0.005 mM or more, more preferably 0.05 mM or more, and still more preferably 0.1 mM or more, and an upper limit thereof is usually 10 mM or less, preferably 5 mM or less, more preferably 2.5 mM or less, and still more preferably 1 mM or less.

An amount of the oxidation catalyst used in the measuring method according to the embodiment of the present invention may vary depending on the type of the used catalyst and the method of measurement and cannot be generalized, but for example, in a case of using peroxidase (POD) as the catalyst and measuring total amount of luminescence, with respect to 25 µL of a sample, the amount of the peroxidase (POD) used is usually 5 µL to 1 mL at a concentration of 0.001 to 1 µmol/L, preferably 10 to 500 µL at a concentration of 0.005 to 0.5 µmol/L and more preferably 20 to 300 µL at a concentration of 0.01 to 0.1 µmol/L. On the other hand, in a case of measuring the amount of luminescence per unit time, with respect to 25 µL of a sample, the amount of the peroxidase (POD) used is usually 5 µL to 1 mL at a concentration of 0.001 to 1 µmol/L, preferably 10 to 500 µL at a concentration of 0.005 to 0.5 µmol/L and more preferably 20 to 300 µL at a concentration of 0.01 to 0.1 µmol/L.

An amount of the oxidizing agent used in the measuring method according to the embodiment of the present invention may vary depending on the type of the used oxidizing agent and the method of measurement and cannot be generalized, but for example, in a case of using hydrogen peroxide as the oxidizing agent and measuring total amount of luminescence, with respect to 25 µL of a sample, the amount of the hydrogen peroxide used is usually 5 µL to 1 mL at a concentration, as a concentration (w/v) of hydrogen peroxide water, of 0.001% to 0.5%, preferably 10 to 500 µL at a concentration of 0.005% to 0.25% and more preferably 20 to 300 µL at a concentration of 0.01% to 0.1%. On the other hand, in a case of measuring the amount of luminescence per unit time, with respect to 25 µL of a sample, the amount of the hydrogen peroxide used is usually 2.5 to 500 µL at a concentration, as a concentration (w/v) of hydrogen peroxide water, of 0.001% to 0.5%, preferably 5 to 250 µL at a concentration of 0.005% to 0.25% and more preferably 10 to 150 µL at a concentration of 0.01% to 0.1%.

An amount of the sensitizer (enhancer) used in the measuring method according to the embodiment of the present invention may vary depending on the type of the used sensitizer (enhancer) and the method of measurement and cannot be generalized, but for example, in a case of using a thiazole derivative as the sensitizer (enhancer) and measuring total amount of luminescence, with respect to 25 µL of a sample, the amount of the thiazole derivative used is usually 5 µL to 1 mL at a concentration of 0.1 µM to 10 mM, preferably 10 to 500 µL at a concentration of 1 µM to 2 mM and more preferably 20 to 300 µL at a concentration of 10 µM to 1 mM. On the other hand, in a case of measuring the amount of luminescence per unit time, with respect to 25 µL of a sample, the amount of the thiazole derivative used is usually 5 µL to 1 mL at a concentration of 0.1 µM to 10 mM, preferably 10 to 500 µL at a concentration of 1 µM to 2 mM and more preferably 20 to 300 µL at a concentration of 10 µM to 1 mM.

In the measuring method according to the embodiment of the present invention, for the purpose of avoiding influence of components other than the target substance contained in the sample, for the purpose of increasing detection sensitivity of the target substance, or the like, an additive used in the related art, such as a surfactant and an activator, may be added to the measurement system. In addition, an amount of these additives added may be appropriately set from a range used in the related art.

The luminescence reaction of the luminescent substrate in the measuring method according to the embodiment of the present invention may be carried out under a condition of usually pH 5 to 12, preferably pH 6 to 11 and more preferably pH 6.5 to 10.5.

Adjustment to such a pH may be carried out by adjusting a pH of a solution such as the luminescent substrate solution, and for example, the pH may be adjusted by using water and/or a buffer solution which dissolves or dilutes the luminescent substrate, the transition metal compound according to the present invention, the oxidation catalyst, or the oxidizing agent. Examples of such a buffer solution include the same one as in specific examples of the buffer solution exemplified in the composition according to the embodiment of the present invention described above, and preferred specific examples thereof are also the same. In addition, the used concentration of the buffer agent contained in the above-described buffer solution is also the same as the above-described range.

In the measuring method according to the embodiment of the present invention, the luminescence reaction of the luminescent substrate and subsequent measurement of the amount of luminescence may be carried out under a temperature condition of usually 10°C to 60°C, preferably 15°C to 50°C and more preferably 20°C to 45°C.

In the measuring method according to the embodiment of the present invention, the time for the luminescence reaction of the luminescent substrate and subsequent luminescence measurement is usually 1 second to 20 minutes, preferably 5 seconds to 15 minutes and more preferably 10 seconds to 10 minutes.

In a case where a labeled antigen or a labeled antibody, labeled with an enzyme such as peroxidase (POD), is used as the oxidation catalyst, an antigen-antibody reaction is performed to form an enzyme-labeled immune complex, and the complex is subjected to the above-described measuring method according to the embodiment of the present invention, the antigen-antibody reaction may be performed under the same pH and temperature conditions as the luminescence reaction of the luminescent substrate in the above-described measuring method.

The reaction time of the above-described antigen-antibody reaction is usually 10 seconds to 120 minutes, preferably 30 seconds to 60 minutes and more preferably 1 to 30 minutes.

The measuring method according to the embodiment of the present invention can be applied to, for example, a measuring method or a detection method using the above-described chemiluminescence reaction in the field of clinical examination, the field of biochemistry, the field of biology, or the like. Examples of such a method include chemiluminescent immunoassay (CLIA), chemiluminescent enzyme immunoassay (CELIA), and electrochemiluminescent immunoassay (ECLEIA), in which an antibody or an antigen, labeled with a luminescent substrate, an oxidation catalyst, or the like, forms an immune complex with the target substance in the sample, and an amount of luminescence of a chemiluminescent substance in the complex is measured. In the measurement of the amount of luminescence, the amount of luminescence of the target substance may be directly or indirectly measured, and the amount of luminescence of a substance (computing substance) which competes with the target substance may be measured.

Specific examples of the measuring method according to the embodiment of the present invention include a measuring method of a target substance, in which, in the presence of the transition metal compound according to the present invention, an antibody (labeled antibody) against the target substance, which is labeled with an oxidation catalyst such as peroxidase (POD), is reacted with the target substance to generate an immune complex of the target substance and the labeled antibody, the immune complex (to which the oxidation catalyst such as POD is coupled) is reacted with hydrogen peroxide and the luminescent substrate, and the amount of luminescence of the resulting chemiluminescent substance is measured.

The above-described target substance is not particularly limited as long as an antibody or an antigen can be obtained therefrom by any methods, and examples thereof include all of the subjects for measurement which are considered measurable by ordinary complement immunoassay methods, such as drugs having biological and clinical importance, metabolites, vitamins, insecticides, steroids, peptides, hormones, hepatitis markers, cancer markers, antibodies, and serum proteins. Specific examples of endocrine function-related materials include thyroid-stimulating hormone (TSH), intact parathyroid hormone (iPTH), growth hormone (GH), somatomedin C (IGF-1), luteinizing hormone (LH), follicle-stimulating hormone (FSH), prolactin (PRL), adrenocorticotropic hormone (ACTH), vasopressin, oxytocin, somatostatin, enkephalin, β-endorphin, thyroxine, triiodothyronine, thyroglobulin, an anti-thyroglobulin antibody, an anti-T3 antibody, an anti-T4 antibody, an anti-TSH antibody, calcitonin, catecholamine, dopamine, serotonin, aldosterone, renin, angiotensin, cortisol, deoxycortisol, cortisone, corticosterone, deoxycorticosterone, androsterone, progesterone, pregnenolone, estrogen, estrone, estriol, estradiol, testosterone, gonadotropin, insulin, an anti-insulin antibody, C-peptide, glucagon, gastrin, secretin, cyclic AMP, cyclic GMP, prostaglandins, thromboxane, erythropoietin, and histamine; specific examples of tumor-related materials include CEA, ferritin, β2-microglobulin, elastase, α-fetoprotein, neuron-specific enolase, a prostatic specific antigen, and CA19-9; specific examples of drug- and vitamin-related materials include phenobarbital, phenytoin, carbamazepine, primidone, ethosuximide, valproic acid, acetazolamide, sulthiame, glutethimide, clonazepam, nitrazepam, diazepam, pentobarbital, secobarbital, bupivacaine, mepivacaine, lidocaine, procainamide, quinidine, digoxin, digitoxin, theophylline, amitriptyline, imipramine, amikacin, gentamicin, tobramycin, cefalexin, sulfamethoxazole, methotrexate, cyclosporin, methyl-prednisolone, salicylic acid, acetaminophen, indomethacin, allopurinol, vitamin A, carotene, vitamin B1, vitamin B2, vitamin B6, vitamin B12, folic acid, vitamin C, vitamin D, and vitamin E; specific examples of serum or plasma protein-related materials include albumin, α1-microglobulin, α1-antitrypsin, α2-macroglobulin, haptoglobulin, hemopexin, transferrin, myoglobin, IgG, IgM, IgA, IgD, IgE, fibrinogen, antithrombin, plasminogen, antiplasmin, protein C, a rheumatoid factor, an anti-DNA antibody, and C reactive protein; and specific examples of virus- and infectious disease-related materials include an HBs antigen, an HBs antibody, an HBc antibody, an HTLV-I antibody, an HTLV-III antibody, TPHA, various virus antigens, and various virus antibodies. Among these target substances, thyroid-stimulating hormone (TSH), intact parathyroid hormone (iPTH), aldosterone, renin, cortisol, an HBs antibody, or an HBc antibody is preferable.

The above-described sample may be derived from a test animal, and examples thereof include biological specimen such as whole blood, serum, blood plasma, urine, saliva, cerebrospinal fluid, tissue fluid, sweat, tears, amniotic fluid, bone marrow fluid, pleural effusion, ascites, indirect fluid, aqueous humor, and vitreous humor.

In the measuring method according to the embodiment of the present invention, a method of measuring the amount of luminescence of thyroid-stimulating hormone (hereinafter, may be abbreviated as TSH) in the sample will be described below as an example.

A sample, a solution containing an anti-thyroid-stimulating hormone antibody (hereinafter, may be abbreviated as an anti-TSH antibody)-coupled magnetic particles, a peroxidase (POD)-labeled anti-TSH antibody solution, a luminol sodium salt, a solution (luminescent substrate solution) containing sodium orthovanadate (V) and 4-(4-hydroxyphenyl)thiazole as a sensitizer (enhancer), a hydrogen peroxide-containing solution are each loaded into an appropriate automatic analyzer provided with a mechanism for keeping a constant atmosphere in a reaction vessel and/or around the reaction vessel in a photometry chamber. Next, the analyzer is started, a sample containing TSH and the solution containing the anti-TSH antibody-coupled magnetic particles are reacted with each other to form an "anti-TSH antibody-coupled magnetic particle-TSH" complex, and B/F separation is performed. Next, a POD-labeled anti-TSH antibody-containing solution is added to and reacted with the solution containing the "anti-TSH antibody-coupled magnetic particle-TSH" complex to form an "anti-TSH antibody-coupled magnetic particle-TSH-POD-labeled anti-TSH antibody" complex, and B/F separation is performed. Subsequently, the luminescent substrate solution and the hydrogen peroxide-containing solution are added to the magnetic particles subjected to the B/F separation, and mixed, and the amount of luminescence derived from TSH contained in the sample may be measured. In addition, as another method, a sample, a solution containing anti-TSH antibody-coupled magnetic particles, a peroxidase (POD)-labeled anti-TSH antibody solution, a luminol sodium salt, a solution (luminescent substrate solution) containing sodium orthovanadate (V) and 4-(4-hydroxyphenyl)thiazole as a sensitizer (enhancer), a hydrogen peroxide-containing solution are each loaded into an appropriate automatic analyzer provided with a mechanism for keeping a constant atmosphere in a reaction vessel and/or around the reaction vessel in a photometry chamber. Next, the analyzer is started, a sample containing TSH, the solution containing the anti-TSH antibody-coupled magnetic particles, and a POD-labeled anti-TSH antibody-containing solution are reacted with each other to form an "anti-TSH antibody-coupled magnetic particle-TSH-POD-labeled anti-TSH antibody" complex, and B/F separation is performed. The luminescent substrate solution and the hydrogen peroxide-containing solution are added to the magnetic particles subjected to the B/F separation, and mixed, and the amount of luminescence derived from TSH contained in the sample may be measured.

The magnetic particles according to the measuring method according to the embodiment of the present invention are not particularly limited as long as they are magnetic particles used in the related art, and commercially available products may be used or those appropriately synthesized by a known method may be used. In addition, the production of the magnetic particles to which an antigen or an antibody is coupled, such as the anti-TSH antibody-coupled magnetic particles formed of the above-described magnetic particles, may be carried out according to a known method.

In a case where the amount of luminescence is measured using a common spectrometer instead of the automatic analyzer, the amount of luminescence derived from the target substance contained in the sample may be measured by reacting a sample, various reagents, and the like by a manual method to perform a luminescence reaction, and then setting a reaction vessel in a spectrometer provided with a mechanism for keeping a constant atmosphere in a reaction vessel and/or around the reaction vessel in a photometry chamber.

As described above, in the measuring method according to the embodiment of the present invention, by coexisting the transition metal compound according to the present invention with the luminescent substrate, the stability of the solution containing the luminescent substrate in a solution state can be improved, and the decrease in amount of luminescence of the luminescent substrate (decrease in measurement sensitivity) can be suppressed. In addition, since the amount of luminescence of the luminescent substrate is unlikely to be adversely affected, there is an effect that the amount of luminescence described from the target substance can be measured with high sensitivity and high accuracy.

### -Kit for measuring luminescence-

The luminescent substrate solution, various reagents, solutions, and the like used in the measuring method according to the embodiment of the present invention may be provided in a form of a kit. Specific examples of such a kit for measuring luminescence include a kit including a reagent containing the transition metal compound according to the present invention, a reagent containing the luminescent substrate, a reagent containing the oxidizing agent, and a reagent containing the oxidation catalyst, and a kit including a reagent containing the transition metal compound according to the present invention and the luminescent substrate, a reagent containing the oxidizing agent, and a reagent containing the oxidation catalyst.

The contents of the luminescent substrate, the transition metal compound according to the present invention, the oxidizing agent, and the oxidation catalyst contained in the above-described kit for measuring luminescence may be set such that, in a case of measuring chemiluminescence, the final concentration and concentration ratio of each component of the luminescent substrate, the transition metal compound according to the present invention, the oxidizing agent, and the oxidation catalyst are within the above-described ranges.

In addition to the above-described reagents, the above-described kit for measuring luminescence may contain an additive used in the related art, such as a sensitizer (enhancer), an activator, a preserving agent, a stabilizer, a preservative, and a surfactant. A content of these additives added may be appropriately set from a range used in the related art.

Examples of the kit for measuring luminescence, containing such an additive and the like, include the following kits.
(i) kit for measuring luminescence, which includes a reagent containing the transition metal compound according to the present invention and the sensitizer (enhancer), a reagent containing the luminescent substrate, a reagent containing the oxidizing agent, and a reagent containing the oxidation catalyst.
(ii) kit for measuring luminescence, which includes a reagent containing the transition metal compound according to the present invention, a reagent containing the luminescent substrate and the sensitizer (enhancer), a reagent containing the oxidizing agent, and a reagent containing the oxidation catalyst.
(iii) kit for measuring luminescence, which includes a reagent containing the transition metal compound according to the present invention, a reagent containing the luminescent substrate, a reagent containing the oxidizing agent and the sensitizer (enhancer), and a reagent containing the oxidation catalyst.
(iv) kit for measuring luminescence, which includes a reagent containing the transition metal compound according to the present invention, a reagent containing the luminescent substrate, a reagent containing the oxidizing agent, a reagent containing the oxidation catalyst, and a reagent containing the sensitizer (enhancer).
(v) kit for measuring luminescence, which includes a reagent containing the transition metal compound according to the present invention, a reagent containing the luminescent substrate, a reagent containing the oxidizing agent, a reagent containing the oxidation catalyst, and a reagent containing the magnetic particles.
(vi) kit for measuring luminescence, which includes a reagent containing the transition metal compound according to the present invention and the sensitizer (enhancer), a reagent containing the luminescent substrate, a reagent containing the oxidizing agent, a reagent containing the oxidation catalyst, and a reagent containing the magnetic particles.
(vii) kit for measuring luminescence, which includes a reagent containing the transition metal compound according to the present invention, a reagent containing the luminescent substrate and the sensitizer (enhancer), a reagent containing the oxidizing agent, a reagent containing the oxidation catalyst, and a reagent containing the magnetic particles.
(viii) kit for measuring luminescence, which includes a reagent containing the transition metal compound according to the present invention, a reagent containing the luminescent substrate, a reagent containing the oxidizing agent and the sensitizer (enhancer), a reagent containing the oxidation catalyst, and a reagent containing the magnetic particles.
(ix) kit for measuring luminescence, which includes a reagent containing the transition metal compound according to the present invention, a reagent containing the luminescent substrate, a reagent containing the oxidizing agent, a reagent containing the oxidation catalyst, a reagent containing the sensitizer, and a reagent containing the magnetic particles.
(x) kit for measuring luminescence, which includes a reagent containing the transition metal compound according to the present invention, the luminescent substrate, and the sensitizer (enhancer), a reagent containing the oxidizing agent, and a reagent containing the oxidation catalyst.
(xi) kit for measuring luminescence, which includes a reagent containing the transition metal compound according to the present invention and the luminescent substrate, a reagent containing the oxidizing agent and the sensitizer (enhancer), and a reagent containing the oxidation catalyst.
(xii) kit for measuring luminescence, which includes a reagent containing the transition metal compound according to the present invention and the luminescent substrate, a reagent containing the oxidizing agent, a reagent containing the oxidation catalyst, and a reagent containing the sensitizer (enhancer).
(xiii) kit for measuring luminescence, which includes a reagent containing the transition metal compound according to the present invention and the luminescent substrate, a reagent containing the oxidizing agent, a reagent containing the oxidation catalyst, and a reagent containing the magnetic particles.
(xiv) kit for measuring luminescence, which includes a reagent containing the transition metal compound according to the present invention, the luminescent substrate, and the sensitizer (enhancer), a reagent containing the oxidizing agent, a reagent containing the oxidation catalyst, and a reagent containing the magnetic particles.
(xv) kit for measuring luminescence, which includes a reagent containing the transition metal compound according to the present invention and the luminescent substrate, a reagent containing the oxidizing agent and the sensitizer (enhancer), a reagent containing the oxidation catalyst, and a reagent containing the magnetic particles.
(xvi) kit for measuring luminescence, which includes a reagent containing the transition metal compound according to the present invention and the luminescent substrate, a reagent containing the oxidizing agent, a reagent containing the oxidation catalyst, a reagent containing the sensitizer (enhancer), and a reagent containing the magnetic particles.

Each reagent in the above-described kit for measuring luminescence may be in any form of a solution state, a frozen state, or a freeze-dried state.

In a case where the chemiluminescence is measured using the above-described kit for measuring luminescence, it is desirable to dissolve or dilute each reagent with water or/and a buffer solution and use the reagent in a solution state. Examples of such a solution (test solution for measuring luminescence) include the following solution.
(i) test solution for measuring luminescence, which includes a luminescent substrate solution containing the transition metal compound according to the present invention and the luminescent substrate, a solution containing the oxidizing agent, and a solution containing the oxidation catalyst.

The contents of the luminescent substrate, the transition metal compound according to the present invention, the oxidizing agent, and the oxidation catalyst contained in the above-described test solution for measuring luminescence may be set such that, in a case of measuring chemiluminescence, the final concentration and concentration ratio of each component of the luminescent substrate, the transition metal compound according to the present invention, the oxidizing agent, and the oxidation catalyst are within the above-described ranges.

In addition to the above-described reagents, the above-described test solution for measuring luminescence may contain an additive used in the related art, such as a sensitizer (enhancer), an activator, a preserving agent, a stabilizer, a preservative, and a surfactant. A content of these additives added may be appropriately set from a range used in the related art.

### -Stabilizer for luminescent substrate-

The present invention also describes a stabilizer for a luminescent substrate, which contains a transition metal compound selected from a vanadium compound, a niobium compound, or a tantalum compound.

In a case of using the stabilizer for a luminescent substrate, the above-described stabilization method according to the embodiment of the present invention can be performed.

Specifically, the transition metal compound can coexist with the luminescent substrate by a method of adding the stabilizer for a luminescent substrate to a reagent containing the luminescent substrate, a method of adding a luminescent substrate or a reagent containing a luminescent substrate to the stabilizer for a luminescent substrate, a method of adding a reagent containing a luminescent substrate and the stabilizer for a luminescent substrate to a dissolving solution such as water and an appropriate buffer solution.

In addition, by measuring the amount of luminescence in the coexistence, the measuring method according to the embodiment of the present invention can be performed.

The stabilizer for a luminescent substrate may be a reagent which is a single substance of the above-described transition metal compound, or may be a reagent containing the above-described transition metal compound and other compounds.

Examples of the other compounds include an additive used in the related art, such as a sensitizer (enhancer), an activator, a preserving agent, a stabilizer, a preservative, and a surfactant.

It is preferable that the stabilizer for a luminescent substrate itself is provided in a form containing no luminescent substrate.

Examples of the stabilizer for a luminescent substrate include the "reagent containing the transition metal compound according to the present invention" and the "reagent containing the transition metal compound according to the present invention and the sensitizer (enhancer)" in the above-described kit.

### Examples

Hereinafter, the present invention will be specifically described based on Examples and Comparative Examples. However, the present invention is not limited to these examples.

### -Preparation of reagents for thyroid-stimulating hormone measurement system using peroxidase (POD) and measurement of thyroid-stimulating hormone-

In a measurement system of thyroid-stimulating hormone using peroxidase (POD), preparation of reagents and measuring method and evaluation method of thyroid-stimulating hormone using the transition metal compound according to the present invention are described below.

### -Example 1-

(1) Preparation of luminescent substrate solution (luminol solution)
   (1-A solution) 398 mg of luminol sodium salt (manufactured by FUJIFII,M Wako Pure Chemical Corporation), 1 mL of dimethyl sulfoxide (DMSO) (manufactured by FUJIFILM Wako Pure Chemical Corporation) solution of 4-(4-hydroxyphenyl)thiazole (manufactured by FUJIFILM Wako Pure Chemical Corporation) with a concentration of 35.4 mg/mL, and 8.77 g of sodium chloride were charged into 1000 mL volumetric flask. Next, 183.9 mg of sodium orthovanadate (V) (manufactured by Santa Cruz Biotechnology, Inc.) was added thereto, a 3-[4-(2-hydroxyethyl)-1-piperazinyl]propanesulfonic acid (EPPS) buffer solution (manufactured by DOJINDO LABORATORIES) was added thereto such that the final concentration was 150 mM, the pH was adjusted to 8.55 with a 5 N sodium hydroxide aqueous solution, and the mixture was uniformly mixed at 25°C to prepare 1000 mL of a luminol solution (luminol concentration: 2 mM, orthovanadic acid (V) concentration: 1 mM, pH: 8.55). The above-described luminol solution was stored at 11°C or 37°C, and a solution 7 days after the storage was used for measurement.
   (1-A' solution) A luminol solution was prepared by the same operation as in (1-A solution), except that the sodium orthovanadate (V) was not added. The above-described luminol solution was stored at 11°C or 37°C, and a solution 7 days after the storage was used for measurement.
(2) Preparation of reagent containing antibody-coupled magnetic particles (reagent of anti-thyroid-stimulating hormone antibody (anti-TSH antibody) (mouse monoclonal antibody)-coupled magnetic particles)
   Magnetic silica particles (produced according to Production Example 1 described in WO2012/173002A) were reacted with 3-aminopropyltriethoxysilane (manufactured by Shin-Etsu Chemical Co., Ltd.) and succinic acid anhydride (manufactured by FUJIFILM Wako Pure Chemical Corporation), the magnetic silica particles were magnetized with a neodymium magnet, and the supernatant was removed to obtain magnetic silica particles having a carboxyl group. Next, the above-described magnetic silica particles were reacted with an anti-TSH antibody (mouse monoclonal antibody) at 26°C for 12 to 16 hours using N-hydroxysuccinic acid imide (manufactured by FUJIFILM Wako Pure Chemical Corporation) and 1-ethyl-3-(3-dimethylaminopropyl)carbodiimide hydrochloride (WSC) (manufactured by DOJINDO LABORATORIES), a blocking solution {0.5% Block Ace (manufactured by Snow Brand Milk Products Co., Ltd.) was added thereto, and the mixture was reacted at 26°C for 12 to 16 hours to produce an anti-TSH antibody (mouse monoclonal antibody)-coupled magnetic particles. The anti-TSH antibody (mouse monoclonal antibody)-coupled magnetic particles were diluted with a 50 mM of 2-(N-morpholino)ethanesulfonic acid (MES) buffer solution (pH: 5.5) to a concentration of 1.0 mg/mL as an anti-TSH antibody-coupled magnetic particle concentration, thereby preparing a reagent containing antibody-coupled magnetic particles (reagent of anti-TSH antibody (mouse monoclonal antibody)-coupled magnetic particles). The reagent was stored in a refrigerator (2°C to 10°C) until use for measurement.
(3) Preparation of buffer solution for immune reaction
   A 50 mM of 2-(N-morpholino)ethanesulfonic acid (MES) buffer solution (pH: 6.0) was prepared to obtain a buffer solution for an immune reaction. The buffer solution was stored in a refrigerator (2°C to 10°C) until use for measurement.
(4) Preparation of enzyme-labeled reagent (peroxidase (POD)-labeled anti-TSH antibody solution)
   According to a method described in the literature (S. YOSHITAKE, M. IMAGAWA, E. ISHIKAWA, H. OGAWA; J Biochem (1982) Vol. 92, (5): 1413-1424), a POD-labeled anti-TSH antibody (mouse monoclonal antibody) was prepared using the anti-TSH antibody (mouse monoclonal antibody) and POD derived from horseradish. The POD-labeled anti-TSH antibody (mouse monoclonal antibody) was diluted with a 50 mM of 2-(N-morpholino)ethanesulfonic acid (MES) buffer solution (pH: 6.5) to a concentration of 0.02 µmol/L as a POD-labeled anti-TSH antibody concentration, thereby preparing an enzyme-labeled reagent (POD-labeled anti-TSH antibody (mouse monoclonal antibody) solution). The reagent was stored in a refrigerator (2°C to 10°C) until use for measurement.
(5) Preparation of hydrogen peroxide aqueous solution
   336 µL hydrogen peroxide water (manufactured by FUJIFILM Wako Pure Chemical Corporation, special reagent, concentration: 30% by weight) was charged into a 1000 mL volumetric flask. Next, the volume of the solution was increased to 1000 mL using distilled water, and the mixture was uniformly mixed at 25°C to prepare a hydrogen peroxide aqueous solution (concentration of hydrogen peroxide (w/v): 0.02%). The hydrogen peroxide aqueous solution was stored in a refrigerator (2°C to 10°C) until use for measurement.
(6) Measurement of chemiluminescence
   Using each of the reagents and solutions obtained in (1) to (5), the amount of luminescence (average value) was measured based on the luminescent substrate solution (1-A solution and 1-A' solution) by the following method, and a stabilizing effect of the luminescent substrate by orthovanadic acid (V) was evaluated.

The antibody-coupled magnetic particles were magnetized from 50 µL of the reagent containing antibody-coupled magnetic particles (reagent of anti-thyroid-stimulating hormone antibody (anti-TSH antibody) (mouse monoclonal antibody)-coupled magnetic particles) with a neodymium magnet, the supernatant was removed, and 50 µL of the buffer solution for an immune reaction was added thereto. Next, 25 µL of a standard TSH solution with a TSH concentration of 0.05 µlU/mL or 0.2 µlU/mL, which had been prepared in 50 mM of 3-(N-morpholino)propanesulfonic acid (MOPS) buffer solution (pH: 7.5), was added thereto in a test tube and mixed, and the mixture was reacted at 37°C for 3 minutes to form an "anti-TSH antibody-coupled magnetic particle-TSH" complex. After the reaction, a cleaning operation in which the magnetic particles were magnetized with a neodymium magnet, the supernatant was removed, 100 µL of physiological saline was added thereto, the magnetic particles were dispersed and magnetized, and the supernatant was removed was performed three times.

Next, 50 µL of the enzyme-labeled reagent (peroxidase (POD)-labeled anti-TSH antibody solution) was added thereto in a test tube, and the mixture was reacted at 37°C for 3 minutes to form an "anti-TSH antibody-coupled magnetic particle-TSH-POD-labeled anti-TSH antibody" complex. After the reaction, a cleaning operation in which the magnetic particles were magnetized with a neodymium magnet, the liquid was aspirated off with an aspirator, 100 µL of physiological saline was added thereto, the magnetic particles were dispersed and magnetized, and the liquid was aspirated off with an aspirator was performed three times.

Next, 100 µL of the luminescent substrate solution (1-A solution or 1-A' solution) and 100 µL of the hydrogen peroxide aqueous solution were simultaneously added thereto, and the amount of luminescence was measured. These reactions and the measurement of the amount of luminescence were performed using a fully automatic chemiluminescent enzyme immunoassay device Accuraseed (registered trademark) (FUJIFILM Wako Pure Chemical Corporation).

The amount of luminescence in a case where the luminescent substrate solution (1-A solution) containing the orthovanadic acid (V) was stored at 11°C and the amount of luminescence after storage at 37°C for 7 days were measured, and the amount of luminescence after storage at 37°C for 7 days with respect to the amount of luminescence at 11°C (ratio of amount of luminescence to 11°C) was calculated. With regard to the luminescent substrate solution (1-A' solution) not containing the orthovanadic acid (V), the amounts of luminescence were measured in the same manner, and the amount of luminescence after storage at 37°C for 7 days with respect to the amount of luminescence at 11°C (ratio of amount of luminescence to 11°C) was calculated. Based on the calculation results, the stabilizing effect of the luminol solution with the presence or absence of orthovanadic acid was evaluated. The results are shown in Table 1.

### -Comparative Example 1-

### (1) Preparation of luminescent substrate solution (luminol solution)

(1-B solution) A luminol solution (luminol concentration: 2 mM, boric acid concentration: 200 mM, pH: 8.55) was prepared by the same operation as in (1-A solution) of Example 1, except that the sodium orthovanadate (V) was not added, and a borate buffer solution was used instead of the EPPS buffer solution. The above-described luminol solution was stored at 11°C or 37°C, and a solution 7 days after the storage was used for measurement.

### (2) Measurement of chemiluminescence

Using the luminol solution obtained in (1) and each of the reagents and solutions obtained in (2) to (5) of Example 1, by the same method as in (6) of Example 1, the amount of luminescence in a case where the luminescent substrate solution (1-B solution) was stored at 11°C and the amount of luminescence after storage at 37°C for 7 days were measured, and the amount of luminescence after storage at 37°C for 7 days with respect to the amount of luminescence at 11°C (ratio of amount of luminescence to 11°C) was calculated. The results are shown in Table 1 together with the results of Example 1.

### -Comparative Example 2-

(1) Preparation of luminescent substrate solution (luminol solution)
   (1-C solution) A luminol solution (luminol concentration: 2 mM, boric acid concentration (w/v): 0.1%, pH: 8.55) was prepared by the same operation as in (1-A solution) of Example 1, except that a boric acid was used instead of the sodium orthovanadate (V). The above-described luminol solution was stored at 11°C or 37°C, and a solution 7 days after the storage was used for measurement.
   (1-C' solution) A luminol solution was prepared by the same operation as in (1-C solution), except that the boric acid was not added. The above-described luminol solution was stored at 11°C or 37°C, and a solution 7 days after the storage was used for measurement.
(2) Measurement of chemiluminescence

Using each luminol solution obtained in (1) and each of the reagents and solutions obtained in (2) to (5) of Example 1, by the same method as in (6) of Example 1, the amount of luminescence in a case where the luminescent substrate solution (1-C solution) containing the boric acid as an additive was stored at 11°C and the amount of luminescence after storage at 37°C for 7 days were measured, and the amount of luminescence after storage at 37°C for 7 days with respect to the amount of luminescence at 11°C (ratio of amount of luminescence to 11°C) was calculated. With regard to the luminescent substrate solution (1-C' solution) not containing the boric acid, the amounts of luminescence were measured in the same manner, and the amount of luminescence after storage at 37°C for 7 days with respect to the amount of luminescence at 11°C (ratio of amount of luminescence to 11°C) was calculated. Based on the calculation results, the stabilizing effect of the luminol solution with the presence or absence of boric acid was evaluated. The results are shown in Table 1 together with the results of Example 1 and Comparative Example 1.

**[Table 1]**

| | | Example 1 | | | | | |
|---|---|---|---|---|---|---|---|
| Buffer solution | | EPPS | | | | | |
| Additive | | Na orthovanadate (-) | | Na orthovanadate (+) | | | |
| Condition | | 11°C | 37°C for 7 days | 11°C | 37°C for 7 days | | |
| Amount of luminescence | 0 µIU/mL | 644 | 534 | 882 | 1038 | | |
| | 0.05 µIU/mL | 11100 | 8016 | 9154 | 8940 | | |
| | 0.2 µIU/mL | 66087 | 49465 | 48606 | 44867 | | |
| Ratio of amount of luminescence to 11°C | 0.05 µIU/mL | | 72.2% | | 97.7% | | |
| | 0.2 µIU/mL | | 74.8% | | 92.3% | | |
| | | | | | | | |

| | | Comparative Example 1 | | Comparative Example 2 | | | |
|---|---|---|---|---|---|---|---|
| Buffer solution | | Boric acid | | EPPS | | | |
| Additive | | | | Boric acid (-) | | Boric acid (+) | |
| Condition | | 11°C | 37°C for 7 days | 11°C | 37°C for 7 days | 11°C | 37°C for 7 days |
| Amount of luminescence | 0 µIU/mL | 1621 | 3435 | 816 | 1047 | 1008 | 1949 |
| | 0.05 µIU/mL | 22781 | 18958 | 12028 | 8047 | 14239 | 11954 |
| Ratio of amount of luminescence to 11°C | 0.05 µIU/mL | | 83.2% | | 66.9% | | 84.0% |

From the results of Example 1, it was found that, in the luminescent substrate solution (composition according to the embodiment of the present invention) in which the sodium orthovanadate (V) (transition metal compound according to the present invention) was added to the luminol (luminescent substrate), compared to the luminescent substrate solution without the sodium orthovanadate (V), a decrease in amount of luminescence (decrease in measurement sensitivity) of the luminescent substrate solution after storage for a long period of time could be suppressed. From this result, it is suggested that, by suppressing decomposition, deterioration, or the like of luminol and stabilizing luminol, the orthovanadic acid (V) can suppress the decrease in amount of luminescence (decrease in measurement sensitivity) of the luminescent substrate. In addition, from the results of Example 1 and Comparative Examples 1 and 2, it was found that, in the luminescent substrate solution (composition according to the embodiment of the present invention) of Example 1 with the sodium orthovanadate (V), compared to the luminescent substrate solution of Comparative Example 1 using the boric acid as a buffer solution or the luminescent substrate solution of Comparative Example 2 using the boric acid as an additive, the stabilizing effect of the luminescent substrate was excellent.

### -Examples 2 and 3 and Comparative Examples 3 and 4-

(1) Preparation of luminescent substrate solution (luminol solution)
   (1-D solution to 1-G solution) Luminol solutions were prepared by the same operation as in (1-A solution) of Example 1, except that the following additive was used instead of the sodium orthovanadate (V). The above-described luminol solution was stored at 11°C or 37°C, and a solution 7 days after the storage was used for measurement.
   1-D solution: sodium metavanadate (V) (concentration: 1 mM)
   1-E solution: ammonium metavanadate (V) (concentration: 1 mM)
   1-F solution: sodium sulfate (concentration: 1 mM)
   1-G solution: lithium chloride (concentration: 1 mM)
   (1-X' solution) A luminol solution was prepared by the same operation as in (1-A' solution) of Example 1. The above-described luminol solution was stored at 11°C or 37°C, and a solution 7 days after the storage was used for measurement.
(2) Measurement of chemiluminescence

Using each luminol solution obtained in (1) and each of the reagents and solutions obtained in (2) to (5) of Example 1, by the same method as in (6) of Example 1, the amount of luminescence in a case where each of the luminescent substrate solutions (1-D solution to 1-G solution) containing various additives was stored at 11°C and the amount of luminescence after storage at 37°C for 7 days were measured, and the amount of luminescence after storage at 37°C for 7 days with respect to the amount of luminescence at 11°C (ratio of amount of luminescence to 11°C) was calculated. With regard to the luminescent substrate solution (1-X' solution) not containing the additive, the amounts of luminescence were measured in the same manner, and the amount of luminescence after storage at 37°C for 7 days with respect to the amount of luminescence at 11°C (ratio of amount of luminescence to 11°C) was calculated. Based on the calculation results, the stabilizing effect of the luminol solution with the presence or absence of various additives was evaluated. The results are shown in Table 2 together with the results of Comparative Examples 1 and 2.

**[Table 2]**

| | | Example 2 | | | | Example 3 | | | |
|---|---|---|---|---|---|---|---|---|---|
| Buffer solution | | EPPS | | | | EPPS | | | |
| Additive | | Na metavanadate (-) | | Na metavanadate (+) | | NH₄ metavanadate (-) | | NH₄ metavanadate (+) | |
| Condition | | 11°C | 37°C for 7 days | 11°C | 37°C for 7 days | 11°C | 37°C for 7 days | 11°C | 37°C for 7 days |
| Amount of luminescence | 0 µIU/mL | 525 | 414 | 588 | 776 | 525 | 414 | 602 | 815 |
| | 0.05 µIU/mL | 10221 | 6520 | 8855 | 7376 | 10221 | 6520 | 8523 | 8304 |
| | 0.2 µIU/mL | 53110 | 37542 | 43390 | 38447 | 53110 | 37542 | 42800 | 43371 |
| Ratio of amount of luminescence to 11°C | 0.05 µIU/mL | | 63.8% | | 83.3% | | 63.8% | | 97.4% |
| | 0.2 µIU/mL | | 70.7% | | 88.6% | | 70.7% | | 101.3% |
| | | | | | | | | | |

| | | Comparative Example 1 | | Comparative Example 2 | | | | | |
|---|---|---|---|---|---|---|---|---|---|
| Buffer solution | | Boric acid | | EPPS | | | | | |
| Additive | | | | Boric acid (-) | | Boric acid (+) | | | |
| Condition | | 11°C | 37°C for 7 days | 11°C | 37°C for 7 days | 11°C | 37°C for 7 days | | |
| Amount of luminescence | 0 µIU/mL | 1621 | 3435 | 816 | 1047 | 1008 | 1949 | | |
| | 0.05 µIU/mL | 22781 | 18958 | 12028 | 8047 | 14239 | 11954 | | |
| Ratio of amount of luminescence to 11°C | 0.05 µIU/mL | | 83.2% | | 66.9% | | 84.0% | | |
| | | | | | | | | | |

| | | Comparative Example 3 | | | | Comparative Example 4 | | | |
|---|---|---|---|---|---|---|---|---|---|
| Buffer solution | | EPPS | | | | EPPS | | | |
| Additive | | Na sulfate (-) | | Na sulfate (+) | | Lithium chloride (-) | | Lithium chloride (+) | |
| Condition | | 11°C | 37°C for 7 days | 11°C | 37°C for 7 days | 11°C | 37°C for 7 days | 11°C | 37°C for 7 days |
| Amount of luminescence | 0 µIU/mL | 525 | 414 | 539 | 445 | 525 | 414 | 504 | 460 |
| | 0.05 µIU/mL | 10221 | 6520 | 10478 | 6581 | 10221 | 6520 | 10109 | 6652 |
| | 0.2 µIU/mL | 53110 | 37542 | 58110 | 38731 | 53110 | 37542 | 56520 | 37670 |
| Ratio of amount of luminescence to 11°C | 0.05 µIU/mL | | 63.8% | | 62.8% | | 63.8% | | 65.8% |
| | 0.2 µIU/mL | | 70.7% | | 66.7% | | 70.7% | | 66.6% |

From the results of Examples 2 and 3, it was found that, in the luminescent substrate solution (composition according to the embodiment of the present invention) in which the sodium metavanadate (V) or ammonium metavanadate (V) (transition metal compound according to the present invention) was added to the luminol (luminescent substrate), compared to the luminescent substrate solution without these additives, a decrease in amount of luminescence (decrease in measurement sensitivity) of the luminescent substrate solution after storage for a long period of time could be suppressed. From this result, it is suggested that not only the orthovanadic acid (V) of Example 1 but also the various transition metal compounds according to the present invention can suppress the decrease in amount of luminescence (decrease in measurement sensitivity) of the luminescent substrate. In addition, from the results of Examples 2 and 3 and Comparative Examples 1 and 2, it was found that, compared to the luminescent substrate solution of Comparative Example 1 using the boric acid as a buffer solution or the luminescent substrate solution of Comparative Example 2 using the boric acid as an additive, the luminescent substrate solutions (composition according to the embodiment of the present invention) of Examples 2 and 3 with the sodium metavanadate (V) or the ammonium metavanadate (V) had an equal or greater stabilizing effect of the luminescent substrate. On the other hand, in a case of using the sodium sulfate, which had a similar structure to the boric acid or the sodium orthovanadate (V) (Comparative Example 3), or a case of using the lithium chloride which dissociated in an aqueous solution to generate ions (Comparative Example 4), the decrease in amount of luminescence of the luminescent substrate solution was observed, and the luminescent substrate could not be stabilized. From these results, since not all compounds which are structurally similar to the boric acid in the related art can contribute to the stabilization of the luminescent substrate, and the luminescent substrate cannot be stabilized using salts such as lithium chloride, it is unexpected that the compound according to the invention can contribute to the stabilization of the luminescent substrate.

### -Examples 4 to 6-

(1) Preparation of luminescent substrate solution (luminol solution)
   (1-H solution to 1-J solution) Luminol solutions were prepared by the same operation as in (1-A solution) of Example 1, except that the following buffer solution was used instead of the EPPS buffer solution. The above-described luminol solution was stored at 11°C or 37°C, and a solution 7 days after the storage was used for measurement.
   1-H solution: piperazine-1,4-bis(2-hydroxy-3-propanesulfonic acid) (POPSO) buffer solution (concentration: 150 mM)
   1-I solution: N-cyclohexyl-2-aminoethanesulfonic acid (CHES) buffer solution (concentration: 150 mM)
   1-J solution: diethanolamine buffer solution (concentration: 150 mM)
   (1-H' solution to 1-J' solution) Luminol solutions were prepared by the same operation as in (1-H solution to 1-J solution), except that the sodium orthovanadate (V) was not added. The above-described luminol solution was stored at 11°C or 37°C, and a solution 7 days after the storage was used for measurement.
(2) Measurement of chemiluminescence

Using each luminol solution obtained in (1) and each of the reagents and solutions obtained in (2) to (5) of Example 1, by the same method as in (6) of Example 1, the amount of luminescence in a case where each of the luminescent substrate solutions (1-H solution to 1-J solution) containing orthovanadic acid (V) and using various buffer solutions was stored at 11°C and the amount of luminescence after storage at 37°C for 7 days were measured, and the amount of luminescence after storage at 37°C for 7 days with respect to the amount of luminescence at 11°C (ratio of amount of luminescence to 11 °C) was calculated. With regard to the luminescent substrate solutions (1-H' solution to 1-J' solution) not containing the orthovanadic acid (V) and using various buffer solutions, the amounts of luminescence were measured in the same manner, and the amount of luminescence after storage at 37°C for 7 days with respect to the amount of luminescence at 11°C (ratio of amount of luminescence to 11°C) was calculated. Based on the calculation results, the stabilizing effect of the luminol solution in a case of using various buffer solutions was evaluated. The results are shown in Table 3 together with the results of Comparative Examples 1 and 2.

**[Table 3]**

| | | Example 4 | | | | Example 5 | | | |
|---|---|---|---|---|---|---|---|---|---|
| Buffer solution | | POPSO | | | | CHES | | | |
| Additive | | Na orthovanadate (-) | | Na orthovanadate (+) | | Na orthovanadate (-) | | Na orthovanadate (+) | |
| Condition | | 11°C | 37°C for 7 days | 11°C | 37°C for 7 days | 11°C | 37°C for 7 days | 11°C | 37°C for 7 days |
| Amount of luminescence | 0 µIU/mL | 791 | 757 | 954 | 948 | 635 | 659 | 888 | 868 |
| | 0.05 µIU/mL | 6241 | 4512 | 6070 | 5029 | 15550 | 11329 | 15669 | 15075 |
| | 0.2 µIU/mL | 34132 | 24945 | 27738 | 23792 | 126518 | 87038 | 111002 | 101782 |
| Ratio of amount of luminescence to 11°C | 0.05 µIU/mL | | | | 82.9% | | | | |
| | 0.2 µIU/mL | | 72.3% 73.1% | | 85.8% | | 72.9% 68.8% | | 962% 91.7% |
| | | | | | | | | | |

| | | Example 6 | | | | | | | |
|---|---|---|---|---|---|---|---|---|---|
| Buffer solution | | Diethanolamine | | | | | | | |
| Additive | | Na orthovanadate (-) | | Na orthovanadate (+) | | | | | |
| Condition | | 11°C | 37°C for 7 days | 11°C | 37°C for 7 days | | | | |
| Amount of luminescence | 0 µIU/mL | 768 | 815 | 2126 | 2853 | | | | |
| | 0.05 µIU/mL | 9637 | 6326 | 13741 | 10547 | | | | |
| | 0.2 µIU/mL | 67059 | 38916 | 74770 | 51901 | | | | |
| Ratio of amount of luminescence to 11°C | 0.05 µIU/mL | | | | 76.8% | | | | |
| | 0.2 µIU/mL | | 65.6% 58.0% | | 69.4% | | | | |
| | | | | | | | | | |

| | | Comparative Example 1 | | Comparative Example 2 | | | | | |
|---|---|---|---|---|---|---|---|---|---|
| Buffer solution | | Boric acid | | EPPS | | | | | |
| Additive | | | | Boric acid (-) | | Boric acid (+) | | | |
| Condition | | 11°C | 37°C for 7 days | 11°C | 37°C for 7 days | 11°C | 37°C for 7 days | | |
| Amount of luminescence | 0 µIU/mL | 1621 | 3435 | 816 | 1047 | 1008 | 1949 | | |
| | 0.05 µIU/mL | 22781 | 18958 | 12028 | 8047 | 14239 | 11954 | | |
| Ratio of amount of luminescence to 11°C | 0.05 µIU/mL | | 83.2% | | 66.9% | | 84.0% | | |

From the results of Examples 4 to 6, it was found that, in the luminescent substrate solution (composition according to the embodiment of the present invention) in which the sodium orthovanadate (V) (transition metal compound according to the present invention) was added to the luminol (luminescent substrate), compared to the luminescent substrate solution without the sodium orthovanadate (V), a decrease in amount of luminescence (decrease in measurement sensitivity) of the luminescent substrate solution after storage for a long period of time could be suppressed, even in a case of using various buffer solutions, regardless of the type of the buffer solution. In addition, from the results of Examples 4 to 6 and Comparative Examples 1 and 2, it was found that, compared to the luminescent substrate solution of Comparative Example 1 using the boric acid as a buffer solution or the luminescent substrate solution of Comparative Example 2 using the boric acid as an additive, the luminescent substrate solutions (composition according to the embodiment of the present invention) of Examples 4 to 6 with the sodium orthovanadate (V) had an equal or greater stabilizing effect of the luminescent substrate.

### -Examples 7 to 9-

(1) Preparation of luminescent substrate solution (luminol solution)
   (1-K solution to 1-M solution) Luminol solutions were prepared by the same operation as in (1-A solution) of Example 1, except that the concentration of the sodium orthovanadate (V) was changed to the concentration shown below. The above-described luminol solution was stored at 11°C or 37°C, and a solution 7 days after the storage was used for measurement.
   1-K solution: concentration of 0.5 mM
   1-L solution: concentration of 1 mM
   1-M solution: concentration of 2.5 mM
   (1-Y' solution) A luminol solution was prepared by the same operation as in (1-A' solution) of Example 1. The above-described luminol solution was stored at 11°C or 37°C, and a solution 7 days after the storage was used for measurement.
(2) Measurement of chemiluminescence

Using each luminol solution obtained in (1) and each of the reagents and solutions obtained in (2) to (5) of Example 1, by the same method as in (6) of Example 1, the amount of luminescence in a case where each of the luminescent substrate solutions (1-K solution to 1-M solution) with various concentrations of the orthovanadic acid (V) was stored at 11°C and the amount of luminescence after storage at 37°C for 7 days were measured, and the amount of luminescence after storage at 37°C for 7 days with respect to the amount of luminescence at 11°C (ratio of amount of luminescence to 11°C) was calculated. With regard to the luminescent substrate solution (1-Y' solution) not containing the orthovanadic acid (V), the amounts of luminescence were measured in the same manner, and the amount of luminescence after storage at 37°C for 7 days with respect to the amount of luminescence at 11°C (ratio of amount of luminescence to 11°C) was calculated. Based on the calculation results, the stabilizing effect of the luminol solution in a case of containing various concentrations of orthovanadic acid (V) was evaluated. The results are shown in Table 4 together with the results of Comparative Examples 1 and 2.

**[Table 4]**

| | | | | Example 7 | | Example 8 | | Example 9 | |
|---|---|---|---|---|---|---|---|---|---|
| Buffer solution | | EPPS | | EPPS | | EPPS | | EPPS | |
| Concentration of Na orthovanadate | | 0 mM (not added) | | | 0.5 mM | 1.0 mM | | 2.5 mM | |
| Condition | | 11°C | 37°C for 7 days | 11°C | 37°C for 7 days | 11°C | 37°C for 7 days | 11°C | 37°C for 7 days |
| Amount of luminescence | 0 µU/mL | 339 | 241 | 581 | 477 | 412 | 489 | 291 | 278 |
| | 0.05 µIU/mL | 7479 | 5540 | 9126 | 8007 | 5959 | 5636 | 2672 | 2420 |
| | 0.2 µIU/mL | 53618 | 40741 | 54009 | 45715 | 34552 | 33124 | 15644 | 15163 |
| Ratio of amount of luminescence to 11°C | 0.05 µIU/mL | | 74.1% | | 87.7% | | 94.6% | | 90.6% |
| | 0.2 µIU/mL | | 76.0% | | 84.6% | | 95.9% | | 96.9% |
| | | | | | | | | | |

| | | Comparative Example 1 | | Comparative Example 2 | | | | | |
|---|---|---|---|---|---|---|---|---|---|
| Buffer solution | | Boric acid | | EPPS | | | | | |
| Additive | | | | Boric acid (-) | | Boric acid (+) | | | |
| Condition | | 11°C | 37°C for 7 days | 11°C | 37°C for 7 days | 11°C | 37°C for 7 days | | |
| Amount of luminescence | 0 µU/mL | 1621 | 3435 | 816 | 1047 | 1008 | 1949 | | |
| | 0.05 µIU/mL | 22781 | 18958 | 12028 | 8047 | 14239 | 11954 | | |
| Ratio of amount of luminescence to 11°C | 0.05 µIU/mL | | 83.2% | | 66.9% | | 84.0% | | |

From the results of Examples 7 to 9, it was found that, in the luminescent substrate solution (composition according to the embodiment of the present invention) in which the sodium orthovanadate (V) (transition metal compound according to the present invention) was added to the luminol (luminescent substrate), compared to the luminescent substrate solution without the sodium orthovanadate (V), a decrease in amount of luminescence (decrease in measurement sensitivity) of the luminescent substrate solution after storage for a long period of time could be suppressed, even in a case of using various concentrations of the orthovanadic acid (V), regardless of the concentration of the orthovanadic acid (V). In addition, from the results of Examples 7 to 9 and Comparative Examples 1 and 2, it was found that, compared to the luminescent substrate solution of Comparative Example 1 using the boric acid as a buffer solution or the luminescent substrate solution of Comparative Example 2 using the boric acid as an additive, the luminescent substrate solutions (composition according to the embodiment of the present invention) of Examples 7 to 9 with the sodium orthovanadate (V) had an equal or greater stabilizing effect of the luminescent substrate.

### -Example 10-

(1) Preparation of luminescent substrate solution (luminol solution)
   (1-N solution) A luminol solution was prepared by the same operation as in (1-A solution) of Example 1, except that the concentration of the EPPS buffer solution was changed to the concentration shown below. The above-described luminol solution was stored at 11°C or 37°C, and a solution 7 days after the storage was used for measurement.
   1-N solution: concentration of 50 mM
   (1-N' solution) A luminol solution was prepared by the same operation as in (1-N solution), except that the sodium orthovanadate (V) was not added. The above-described luminol solution was stored at 11°C or 37°C, and a solution 7 days after the storage was used for measurement.
(2) Measurement of chemiluminescence

Using each luminol solution obtained in (1) and each of the reagents and solutions obtained in (2) to (5) of Example 1, by the same method as in (6) of Example 1, the amount of luminescence in a case where the luminescent substrate solution (1-N solution) with a concentration of the EPPS buffer solution of 50 mM was stored at 11°C and the amount of luminescence after storage at 37°C for 7 days were measured, and the amount of luminescence after storage at 37°C for 7 days with respect to the amount of luminescence at 11°C (ratio of amount of luminescence to 11°C) was calculated. With regard to the luminescent substrate solution (1-N' solution) not containing the orthovanadic acid (V), the amounts of luminescence were measured in the same manner, and the amount of luminescence after storage at 37°C for 7 days with respect to the amount of luminescence at 11°C (ratio of amount of luminescence to 11°C) was calculated. Based on the calculation results, the stabilizing effect of the luminol solution in a case of containing the orthovanadic acid (V) was evaluated. The results are shown in Table 5 together with the results of Example 1 and Comparative Examples 1 and 2.

**[Table 5]**

| | | Example 1 | | | | Example 10 | | | |
|---|---|---|---|---|---|---|---|---|---|
| Buffer solution | | EPPS 150 mM | | | | EPPS 50 mM | | | |
| Additive | | Na orthovanadate (-) | | Na orthovanadate (+) | | Na orthovanadate (-) | | Na orthovanadate (+) | |
| Condition | | 11°C | 37°C for 7 days | 11°C | 37°C for 7 days | 11°C | 37°C for 7 days | 11°C | 37°C for 7 days |
| Amount of luminescence | 0 µIU/mL | 644 | 534 | 882 | 1038 | 611 | 1047 | 1008 | 1949 |
| | 0.05 µIU/mL | 11100 | 8016 | 9154 | 8940 | 16217 | 10803 | 14459 | 11818 |
| Ratio of amount of luminescence to 11°C | 0.05 µIU/mL | | 72.2% | | 97.7% | | 66.6% | | 81.7% |
| | | | | | | | | | |

| | | Comparative Example 1 | | Comparative Example 2 | | | | | |
|---|---|---|---|---|---|---|---|---|---|
| Buffer solution | | Boric acid | | EPPS | | | | | |
| Additive | | | | Boric acid (-) | | Boric acid (+) | | | |
| Condition | | 11°C | 37°C for 7 days | 11°C | 37°C for 7 days | 11°C | 37°C for 7 days | | |
| Amount of luminescence | 0 µIU/mL | 1621 | 3435 | 816 | 1047 | 1008 | 1949 | | |
| | 0.05 µIU/mL | 22781 | 18958 | 12028 | 8047 | 14239 | 11954 | | |
| Ratio of amount of luminescence to 11°C | 0.05 µIU/mL | | 83.2% | | 66.9% | | 84.0% | | |

From the results of Examples 1 and 10, it was found that, in the luminescent substrate solution (composition according to the embodiment of the present invention) in which the sodium orthovanadate (V) (transition metal compound according to the present invention) was added to the luminol (luminescent substrate), compared to the luminescent substrate solution without the sodium orthovanadate (V), a decrease in amount of luminescence (decrease in measurement sensitivity) of the luminescent substrate solution after storage for a long period of time could be suppressed, even in a case of using buffer solutions with various concentrations, regardless of the concentration of the buffer solution. In addition, from the results of Example 10 and Comparative Examples 1 and 2, it was found that, compared to the luminescent substrate solution of Comparative Example 1 using the boric acid as a buffer solution or the luminescent substrate solution of Comparative Example 2 using the boric acid as an additive, the luminescent substrate solution (composition according to the embodiment of the present invention) of Example 10 with the sodium orthovanadate (V) had an equal or greater stabilizing effect of the luminescent substrate.

### -Examples 11 to 13-

### (1) Preparation of luminescent substrate solution (L-012 solution)

Example 11: (1-O solution) 156 mg of 8-amino-5-chloro-7-phenylpyrido[3,4-d]pyridazine-1,4(2H,3H)-dione (L-012) sodium salt (manufactured by FUJIFII,M Wako Pure Chemical Corporation), and 1 mL of dimethyl sulfoxide (DMSO) (manufactured by FUJIFILM Wako Pure Chemical Corporation) solution of 4-(4-hydroxyphenyl)thiazole (manufactured by FUJIFILM Wako Pure Chemical Corporation) with a concentration of 35.4 mg/mL were charged into 1000 mL volumetric flask. Next, 183.9 mg of sodium orthovanadate (V) (manufactured by Santa Cruz Biotechnology, Inc.) was added thereto, an EPPS buffer solution (manufactured by DOJINDO LABORATORIES) was added thereto such that the final concentration was 50 mM, the pH was adjusted to 8.00 with a 5 N sodium hydroxide aqueous solution, and the mixture was uniformly mixed at 25°C to prepare 1000 mL of an L-012 solution (L-012 concentration: 0.5 mM, orthovanadic acid (V) concentration: 1 mM, pH: 8.00). The above-described L-012 solution was stored at 11°C or 37°C, and a solution 7 days after the storage was used for measurement.

Examples 12 and 13: (1-P solution and 1-Q solution) L-012 solutions were prepared by the same operation as in (1-O solution) of Example 11, except that the concentration of the sodium orthovanadate (V) was changed to the concentration shown below. The above-described L-012 solution was stored at 11°C or 37°C, and a solution 7 days after the storage was used for measurement.
1-P solution: concentration of 0.5 mM
1-Q solution: concentration of 2.5 mM
(1-Z' solution) An L-012 solution was prepared by the same operation as in (1-O solution), except that the sodium orthovanadate (V) was not added.

### (6) Measurement of chemiluminescence

Using each L-012 solution obtained in (1) and each of the reagents and solutions obtained in (2) to (5) of Example 1, by the same method as in (6) of Example 1, the amount of luminescence in a case where each of the luminescent substrate solutions (1-O solution to 1-Q solution) with various concentrations of the orthovanadic acid (V) was stored at 11°C and the amount of luminescence after storage at 37°C for 7 days were measured, and the amount of luminescence after storage at 37°C for 7 days with respect to the amount of luminescence at 11°C (ratio of amount of luminescence to 11°C) was calculated. With regard to the luminescent substrate solution (1-Z' solution) not containing the orthovanadic acid (V), the amounts of luminescence were measured in the same manner, and the amount of luminescence after storage at 37°C for 7 days with respect to the amount of luminescence at 11°C (ratio of amount of luminescence to 11°C) was calculated. Based on the calculation results, the stabilizing effect of the L-012 solution in a case of containing various concentrations of orthovanadic acid (V) was evaluated. The results are shown in Table 6.

**[Table 6]**

| | | | | Example 11 | | Example 12 | | Example 13 | |
|---|---|---|---|---|---|---|---|---|---|
| Buffer solution | | EPPS | | EPPS | | EPPS | | EPPS | |
| Concentration of Na orthovanadate | | 0 mM (not added) | | 1 mM | | 0.5 mM | | 2.5 mM | |
| Condition | | 11°C | 37°C for 7 days | 11°C | 37°C for 7 days | 11°C | 37°C for 7 days | 11°C | 37°C for 7 days |
| Amount of luminescence | 0 µIU/mL | 8170 | 2801 | 5256 | 3532 | 4679 | 3806 | 2440 | 2295 |
| | 0.05 µIU/mL | 159155 | 129649 | 135612 | 125408 | 147953 | 143969 | 115842 | 106757 |
| | 0.2 µIU/mL | 1025192 | 898791 | 805804 | 819352 | 958174 | 930996 | 777759 | 719618 |
| Ratio of amount of luminescence to 11°C | 0.05 µIU/mL | | 81.5% | | 92.5% | | 97.3% | | 92.2% |
| | 0.2 µIU/mL | | 87.7% | | 101.7% | | 97.2% | | 92.5% |

From the results of Example 11, it was found that, in the luminescent substrate solution (composition according to the embodiment of the present invention) in which the sodium orthovanadate (V) (transition metal compound according to the present invention) was added to the L-012, compared to the luminescent substrate solution without the sodium orthovanadate (V), a decrease in amount of luminescence (decrease in measurement sensitivity) of the luminescent substrate solution after storage for a long period of time could be suppressed, even in a case where the L-012 was used instead of the luminol as the luminescent substrate. From this result, it is suggested that, by suppressing decomposition, deterioration, or the like of not only the luminol but also various luminescent substrates such as L-012 and stabilizing the luminescent substrate, the orthovanadic acid (V) can suppress the decrease in amount of luminescence (decrease in measurement sensitivity) of the luminescent substrate. In addition, from the results of Examples 12 and 13, it was found that the stabilizing effect of L-012 was excellent even in a case of using various concentrations of the orthovanadic acid (V), regardless of the concentration of the orthovanadic acid (V).

From the above results, in the luminescent substrate solution (composition according to the embodiment of the present invention) containing the transition metal compound according to the present invention, it was found that the stability of the luminescent substrate in a solution state could be improved, so that, in chemiluminescence measurement, it is possible to suppress the decrease in amount of luminescence (decrease in measurement sensitivity) of the luminescence substrate due to the decomposition, deterioration, or the like of the luminescence substrate, and to detect and measure the target substance with high accuracy.

In the composition and stabilization method according to the embodiment of the present invention, the stability of the luminescent substrate can be improved by coexisting the transition metal compound selected from a vanadium compound, a niobium compound, or a tantalum compound with the luminescent substrate. As a result, it is possible to suppress the decrease in amount of luminescence (decrease in measurement sensitivity) of the luminescent substrate due to the decomposition, deterioration, or the like of the luminescent substrate, so that, for example, in chemiluminescence measurement, by using the composition and the like according to the embodiment of the present invention, a target substance in a living body can be measured with high sensitivity and high accuracy.

## Claims

1. A composition comprising:
a transition metal compound selected from a vanadium compound, a niobium compound, or a tantalum compound; and
a luminescent substrate.

2. The composition according to claim 1,
wherein the transition metal compound is a vanadium compound.

3. The composition according to claim 1,
wherein the transition metal compound is a compound represented by General Formula (1) or General Formula (2), 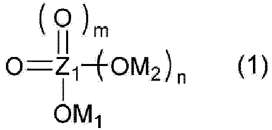 (in General Formula (1), Z₁ represents a vanadium atom, a niobium atom, or a tantalum atom, M₁ represents a hydrogen atom, an alkali metal atom, or NH₄, M₂'s each independently represent a hydrogen atom, an alkali metal atom, or NH₄, m represents 0 or 1, and n represents 2 in a case where m is 0 or represents 0 in a case where m is 1) 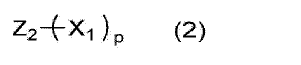 (in General Formula (2), Z₂ represents a vanadium atom, a niobium atom, or a tantalum atom, X₁ represents a hydroxy group, a chlorine atom, or a bromine atom, and p represents an integer of 2 to 5).

4. The composition according to claim 3,
wherein the composition contains the compound represented by General Formula (1) and a luminescent substrate.

5. The composition according to claim 4,
wherein Z₁ in General Formula (1) is a vanadium atom.

6. The composition according to claim 4,
wherein the compound represented by General Formula (1) is orthovanadic acid, an alkali metal salt of orthovanadic acid, an ammonium salt of orthovanadic acid, metavanadic acid, an alkali metal salt of metavanadic acid, or an ammonium salt of metavanadic acid.

7. The composition according to claim 1,
wherein the luminescent substrate is luminols, a chemically acceptable salt of luminols, 8-amino-5-chloro-7-phenylpyrido[3,4-d]pyridazine-1,4(2H,3H)-dione, or a chemically acceptable salt of 8-amino-5-chloro-7-phenylpyrido[3,4-d]pyridazine-1,4(2H,3H)-dione.

8. The composition according to claim 1,
wherein the transition metal compound is a compound for stabilizing the luminescent substrate.

9. The composition according to claim 1,
wherein a concentration of the transition metal compound in the composition is 0.01 to 10 mM.

10. The composition according to claim 1,
wherein a pH of the composition is 5 to 12.

11. A stabilization method of a luminescent substrate, comprising:
coexisting a transition metal compound selected from a vanadium compound, a niobium compound, or a tantalum compound with a luminescent substrate.

12. The stabilization method according to claim 11,
wherein the transition metal compound is added to a solution containing the luminescent substrate for the coexistence.

13. The stabilization method according to claim 11,
wherein the transition metal compound is a compound represented by General Formula (1) or General Formula (2), 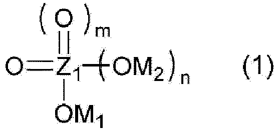 (in General Formula (1), Z₁ represents a vanadium atom, a niobium atom, or a tantalum atom, M₁ represents a hydrogen atom, an alkali metal atom, or NH₄, M₂'s each independently represent a hydrogen atom, an alkali metal atom, or NH₄, m represents 0 or 1, and n represents 2 in a case where m is 0 or represents 0 in a case where m is 1) 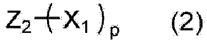 (in General Formula (2), Z₂ represents a vanadium atom, a niobium atom, or a tantalum atom, X₁ represents a hydroxy group, a chlorine atom, or a bromine atom, and p represents an integer of 2 to 5).

14. A method for measuring an amount of luminescence, the method comprising:
reacting a luminescent substrate with an oxidizing agent and an oxidation catalyst in a presence of a transition metal compound selected from a vanadium compound, a niobium compound, or a tantalum compound.

15. The method according to claim 14,
wherein the transition metal compound is a compound represented by General Formula (1) or General Formula (2), 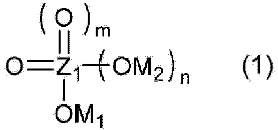 (in General Formula (1), Z₁ represents a vanadium atom, a niobium atom, or a tantalum atom, M₁ represents a hydrogen atom, an alkali metal atom, or NH₄, M₂'s each independently represent a hydrogen atom, an alkali metal atom, or NH₄, m represents 0 or 1, and n represents 2 in a case where m is 0 or represents 0 in a case where m is 1) 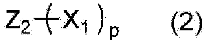 (in General Formula (2), Z₂ represents a vanadium atom, a niobium atom, or a tantalum atom, X₁ represents a hydroxy group, a chlorine atom, or a bromine atom, and p represents an integer of 2 to 5).

16. A stabilizer for a luminescent substrate, comprising:
a transition metal compound selected from a vanadium compound, a niobium compound, or a tantalum compound.
